# EUROPEAN PATENT SPECIFICATION

(11) **EP 1 155 121 B1**
(45) Date of publication and mention of the grant of the patent: **26.04.2006**
(21) Application number: 00910316.9
(22) Date of filing: 23.02.2000
(51) Int. Cl.: A61K 31/164, A61K 31/22, A61K 31/366, A61K 31/40, A61K 31/405, G01N 33/92, A61P 3/06

(54) **COMPOSITIONS AND METHODS FOR MODULATING SERUM CHOLESTEROL**
ZUSAMMENSETZUNGEN UND VERFAHREN ZUR REGULIERUNG DES SERUMCHOLESTERINS
COMPOSITIONS ET PROCEDES POUR MODULER LE CHOLESTEROL SERIQUE

(30) Priority: 24.02.1999 US 121447 P
(43) Date of publication of application: 21.11.2001
(73) Proprietor: THE JOHNS HOPKINS UNIVERSITY, Baltimore, MD 21205-2109 (US)
(72) Inventor: CHATTERJEE, Subroto, Columbia, MD 20144 (US)
(74) Representative: Johansen, Marianne
(86) International application number: PCT/US2000/004657
(87) International publication number: WO 2000/050574

(56) References cited:
- FR-A- 2 747 307
- US-A- 3 876 789
- US-A- 5 498 696
- LAWLER JOSEPH F JR ET AL: "Tumor necrosis factor-alpha stimulates the maturation of sterol regulatory element binding protein-1 in human hepatocytes through the action of neutral sphingomyelinase." JOURNAL OF BIOLOGICAL CHEMISTRY, vol. 273, no. 9, 27 February 1998 (1998-02-27), pages 5053-5059, XP002232365 ISSN: 0021-9258
- CHATTERJEE SUBROTO: "Neutral sphingomyelinase: Past, present and future." CHEMISTRY AND PHYSICS OF LIPIDS, vol. 102, no. 1-2, November 1999 (1999-11), pages 79-96, XP002232366 ISSN: 0009-3084
- FRISHMAN ET AL.: 'Lovastatin: An HMG-CoA reductase inhibitor for lowering cholesterol' MEDICAL CLINICS OF NORTH AMERICA, vol. 73, no. 2, March 1989, pages 437 - 448, XP002928774
- DUANE W.C. ET AL.: 'Simvastatin, a competitive inhibitor of HMG coenzyme A reductase lowers cholesterol saturation index of gall bladder bile' HEPATOLOGY, vol. 8, no. 5, 1988, pages 1147 - 1150, XP002928776
- MITCHELL J.C. ET AL.: 'Effects of lovastatin on biliary lipid secretion and bile acid metabolism in humans' J. LIPID RES., vol. 32, 1991, pages 71 - 78, XP002928775

### SEQUENCE LISTING

<110> Johns Hopkins University
   Chatterjee, Subroto
<120> Compositions and methods for modulating serum cholesterol
<130> P00012 EP01
<140> 00910316.9-2105/1155121
   <141> 2000-02-23
<150> PCT/US00/04657
   <151> 2000-02-23
<160> 2
<170> PatentIn version 3.1
<210> 1
   <211> 1197
   <212> DNA
   <213>
<220>
   <221> CDS
   <222> (1)..(1197)
   <223>
<400> 1
   atg atg aca tat cac gaa acg cgc gcg ttg gct caa agc gac tta cag      48
   caa ctc tat gcg gca ctt gaa aca act gaa ttt ggc gct tac ttt gcg      96
   aca ccc gct gat gat act tta cgt ttt ggc att ggc gca atc gct acg     144
   gca aaa acg gct cag gca tta caa ggt gcg gtt gtt ttt ggt gcg cag     192
   tca ttt gat gaa caa gag tac ccg cag tct gaa ttg atg gcg ggt ttt     240
   tgg ttt gtc ccc gaa gtg atg gtg acc atc gcg gca gat aaa atc acg     288
   ttc gga tca gat acc gta tct gat ttt acg acg tgg ctg gcg cag ttc     336
   gtg cca aaa cag cca aat acg gtg acc act agt cat gtg aca gat gaa     384
   gtg gat tgg atc gaa cgg aca gag aat ttg att gat acc tta gcc atc     432
   gat caa acc tta gcc aaa gtc gtt ttt ggt cgg caa cag acc ctg cag     480
   tta tcc gac acg tta cga ctg gca caa att att cgt gcg tta gct gag     528
   cag gcg aat acg tat cat gtg gtt tta aag cga cat gat gaa ttg ttt     576
   att tca gca aca ccg gaa cgg tta gtg gct atg tca ggt ggt cag atc     624
   gct acg gcg gcg gtc gct ggg aca agc cgg cgc ggg acg gat ggc gct     672
   gac gat atc gcg tta ggc gaa gcg ttg tta gcc agt cag aaa aac cgc     720
   att gaa cat caa tat gtc gtg gca agt atc acg aca cgc ttg caa gac     768
   gtg acg acg tcg cta aag gtg ccg gcc atg cca agt tta ctc aaa aat     816
   aag caa gtt cag cat ttg tac aca cca att aca ggg gac att gcg gca     864
   cat tta agt gtg acc gcg att gtt gac cgc ttg cat cca aca cca gca     912
   ctg ggt ggc gtc cca cgt gaa gcg gcc ctg tat tac att gcg acc cat     960
   gag aag aca cct cgt ggc ttg ttt gca ggt cct att ggc tat ttt acc      1008
   gca gat aat agt ggg gaa ttt gtg gtt ggc atc cgt tcc atg tat gtg      1056
   aat caa acg cag cga cga gca act tta ttt gct ggt gcc ggg att gtg      1104
   gct gac tcc gat gcg caa caa gaa tat gaa gaa act ggg ttg aaa ttt      1152
   gaa ccc atg cgg caa ttg tta aag gac tac aat cat gtc gaa tga          1197
<210> 2
   <211> 398
   <212> PRT
   <213>
<400> 2

## Description

### FIELD OF THE INVENTION

The present invention relates to compositions and uses of the compositions for preparing pharmaceutical compositions for modulating serum cholesterol. In one aspect, the invention features novel anti-lipemic drugs that include at least one identified effector, a ceramide of the Low Density Lipoprotein (LDL) receptor and at least one identified serum cholesterol inhibitor. In a particular aspect, the anti-lipemic drug includes a ceramide linked to the serum cholesterol inhibitor. Additionally provided are uses as described above for significantly stabilizing or reducing serum cholesterol levels in a subject mammal and particularly a human patient.

### BACKGROUND OF THE INVENTION

There is nearly universal agreement that cholesterol is a key lipid constituent of cell membranes. Cholesterol is generally understood to be essential for normal growth and viability of most higher organisms. Too much serum cholesterol has been correlated with life threatening lipid related diseases including hyperlipoproteinemia, stoke, coronary heart disease, and especially artherosclerosis and related conditions. See generally Stryer, L. (1988) in *Biochemistry*, 3^{rd} Ed. W.H. Freeman and Co. New York, pp. 547-574; and Brown, M.S. and Goldstein, J.L. (1993) in *The Pharmacological Basis of Therapuetics* (8^{th} Ed.) Gilman, A.G. et al. eds. McGraw-Hill/New York, pp. 874-896.

The regulation of serum cholesterol in mammals and particularly primates has attracted significant attention. It is often reported that regulation of cholesterol homeostasis in humans and other mammals involves regulation of cholesterol production, bile acid biosynthesis and catabolism of specific serum cholesterol carriers. Important serum cholesterol carriers are called LDL (low density lipoprotein) particles. The LDL receptor has been reported to facilitate intemalization of the LDL particle into those cells in need of cholesterol. See e.g., Brown, M.S. and Goldstein, J.L. (1986) *Science* 232: 34-47; and Goldstein, J.L. and Brown, (1986) *Nature*, 348; 425; and references cited therein.

The LDL receptor has been disclosed as impacting serum cholesterol levels in humans. For example, there has been recognition that cells with enough cholesterol do not make sufficient LDL receptors, thereby reducing or even blocking uptake of cholesterol by the cell. In this instance, serum cholesterol levels rise substantially which can contribute to the development or severity of disease. Conversely, cells in need of cholesterol often have capacity to make more LDL receptors, thereby facilitating a decrease in serum cholesterol. Accordingly, there has been specific attention focused on regulating the LDL receptor as one therapeutic approach for stabilizing or reducing serum cholesterol levels in human patients.

In particular, it has been reported that transcription of the LDL receptor gene is suppressed when sterols accumulate and induced when sterols are depleted. Sterol sensitivity is thought to be conferred by a 10 bascpair (bp) sequence upstream of the LDLr gene known as the sterol regulatory element (SRE). It has been disclosed that the mature form of the sterol regulatory element binding protein-1 (SREBP-1) binds to the SRE and promotes transcription.

There have been additional reports that the activity of SREBP-1 is influenced by sterol induced proteolysis. There is recognition that the SREBP-1 proteolysis is impacted in some settings by a cell receptor termed "cytokine tumor necrosis factor" (TNF-α).

In particular, the TNF-α receptor has been reported to influence a wide range of biological effects. However, the TNF-α receptor remains incompletely characterized. Elucidation of TNF-α pathways is sometimes complicated by presence of at least two TNF receptors. The receptors share some common downstream effectors but also signal via receptor specific pathways. See the references cited below for additional disclosure relating to the TNF-α receptor.

There has been understanding that one consequence of TNF signaling is the activation of neutral sphingomyelinase (N-SMase). Neutral sphingomyelinase is a membrane bound enzyme that catalyzes the hydrolysis of sphingomyelin to ceramide and phosphocholine at a pH optima of 7.4. The role of neutral sphingomyelinase in signal transduction has primarily been related to ability to generate the lipid second messenger ceramide. In addition to TNF-α, Fas receptor ligand, vitamin D₃, interleukin-1β, nerve growth factor, anti-CD28 antibodies and γ-interferon have all been shown to increase ceramide levels.

In particular, sphingomyelinases type-C (E.C. 3.1.4.12) are a group of phospholipases that catalyze the hydrolytic cleavage of sphingomyelin via the following reaction (1).

Sphingomyelin → Ceramide + Phosphocholine (1)

See S. Chatterjee, *Adv. Lipid Res*., 26:25-48 (1993); S. Chatterjee et al., *J. Biol Chem.,* 264:12,534-12,561 (1989); and S. Chatterjee et al., *Methods in Enzymology, Phospholipase,* 197:540-547 (1991).

In addition to the biological roles of sphingomyelin and ceramide in signal transduction pathways involving cell regulation, more recent evidence has emerged suggesting that sphingomyclinases may be involved in cholesterol homeostasis and paiticularty induction of LDL receptor activity. See S. Chatterjee, *Advances in Lipid Research,* 26:25-48 (1993). Additional work supports a possible role of ceramide in programmed cell death and/or "apoptosis" and activation of the gene for nuclear factor (NF)-kB. See A. Alessenko and S. Chatterjee, *Mol. Cell. Biochem*., 143:169 (1995).

In the french patent application No. 2 747 307 is suggested the use of ceramide alcone or in combination with a metal or vitamin E for treatment of diseases associated with a high cholesterol level.

It has been suggested that certain enzymes involved in making cholesterol exert a significant effect on cholesterol homeostasis. Accordingly, there has been substantial interest in identifying drugs with capacity to modulate these enzymes especially to stabilize or reduce serum cholesterol to tolerable ranges. Illustrative agents include commercially available serum cholesterol inhibitors such as fluvastatin, simvastatin, lovastatin, pravastatin, and atorvastatin. See Brown, M.S. and Goldstein, J.L. (1993), *supra* for additional disclosure relating to these and other agents such as mevinolin (compactin).

Experiments wherein the serum cholesterol inhibitor lovastatin is combined with other agents known to lower cholesterol, such as the bile acid binding resin, colestipol, and neomycin, have also been performed. See Frishman, W.H., and Rapier, R.C., (1989) *Med. Clinics. of North America* 73: 437-448.

Although some clinical benefit has been reported to follow use of these and other serum lowering agents, there have been reports of significant side-effects. See e.g., Brown, M.S. and Goldstein, J.L. (1993), *supra*; and *Physicians' Desk Reference* 1997 (51^{st} ed.) Medical Economics Co. Accordingly, there is a need to have drugs that exhibit more desirable charactenstics such as enhanced potency and better patient tolerance. There is a specific need to reduce levels of administered cholesterol lowering agents for some patients.

There is also a need to identify drugs that can modulate the SREBP-1 protein and especially the LDL receptor. Moreover, methods for identifying pharmacological drugs of interest by automated, high throughput drug screening have become increasing relied upon in a variety of pharmaceutical and biotechnology drug development programs. Unfortunately, requisite drugs for such high throughput screening assays are not widespread. A significant reason for lack of progress in this area is insufficient understanding of molecules (i.e. effectors) that impact SREP-1 and the LDL receptor.

It thus would be desirable to have anti-lipemic drugs with dual capacity to modulate the LDL receptor and serum cholesterol levels. It would be particularly desirable if such anti-lipemic drugs could be administered to subject mammal at doses near or below those presently used with many serum cholesterol inhibitors. It would be further desirable to have effective in vitro and in vivo assays for identifying drugs with potential to modulate the LDL receptor particularly involving SREP-1 protein maturation.

### SUMMARY OF THE INVENTION

The present invention generally relates to compositions and uses of compositions for preparly pharmaceutical compositions for modulating serum cholesterol in a subject mammal more specific, the invention relates to an anti-lipemic drug comprising ceramide, which is an effecter of the sterol regulatory element binding protein (SREBP-1), and a serum cholesterol inhibitor, which is an inhibitor of HMG CoA reductase. In a particular aspect, the drugs include one identified ceramide linked to one identified serum cholesterol inhibitor. Additionally provided are methods for identifying anti-lipemic drugs capable of modulating the LDL receptor and specifically SREBP-1 maturation, including assays designed to identify pharmacological drugs capable of stabilizing or reducing serum cholesterol levels in a mammal and particularly a human patient. Accordingly, in a specific aspect, the invention relates to an *ex vivo* method for determining therapeutic capacity of an anti-lipemic drug as defined above for treating a cholesterol related disease in a mammal, the method comprising:
a) providing a population of cells capable of expressing SREBP-1,
b) contacting the cells with the anti-lipemic drug in an amount sufficient to induce maturation of the SREBP-1,
c) culturing the cells in medium; and
d) detecting maturation of the SREBP-1 as indicative of the therapeutic capacity of the anti-lipemic drug in treating the disease.

We have discovered a spectrum of compositions and methods for treating or preventing disorders modulated by cholesterol Sometimes the disorders will be referred to herein as "cholesterol related disorders" or a similar term. More specifically, we have identified anti-lipemic drugs that include at least one identified ceramide and at least one identified serum cholesterol inhibitor. Particular anti-lipemic drugs of this invention usually have one of each component although drugs having multiple effectors and inhibitors (e.g., between from about 2 to 5 of each) are contemplated. Preferred anti-upcmic drugs feature specifically defined charactenstics such as capacity to stabilize or reduce serum cholesterol levels in a subject mammal as determined by in vitro or in vivo assays described below.

More specifically, the present invention provides a variety of specific anti-lipemic drugs and use of same for the preparation of a phamaceutical composition for modulating serum cholesterol level in a mammal, and/or for treating a disorder in a mammal associated with high serum cholesterol levels. Illustrative disorders are known in the field and include hyperlipoproteinemia including hypercholesterolemia, stroke, obesity, compulsive eating disorders, cardiac disease including atherosclerosis, cerebral atherosclerosis, cholesteryl ester storage disorder, liver disease including organ transplantation failure and cirrhosis; diseases of the biliary system, and viral infection, particularly those infections facilitating encephalitis or related disorders.

Particular anti-lipemic drugs in accord with this invention include ceramide, which is a SREBP-1 effector and one synthetic or semi-synthetic statin as inhibitor of 3-hydroxy-3-methylglutaryl (HMG) CoA reductase.

The ceramide molecule is preferably naturally-occurring (ie. can be isolated in substantially pure form from a biological source). A more preferred ceramide for use in the drug is any one of C-2, C-4, C-6 or C-8 ceramide. In a preferred embodiment the ceramide is C-2 ceramide.

The anti-lipernic drug further includes a statin as inhibitor of HMG CoA reductase. It is generally preferred that the effector and the inhibitor are be combined in a way to facilitate function for which the drug was intended. A preferred function is to stabilize or reduce serum cholesterol as determined by a conventional in vivo assays defined below. In most instances, covalent attachment between the effector and the inhibitor will be preferred although other associations will be suitable for some applications. Preferred cholesterol inhibitors have recognized capacity to inhibit the redactase, thereby lowering serum cholesterol. Illustrative inhibitors include commercially available serum cholesterol inhibitors acceptable for human use, e.g., fluvastatin, simvastatin, lovastatin, pravastatin, mevinolin (compactin), atorvastatin; or a clinically acceptable derivative thereof The effector ceramide is preferably associated with the inhibitor ofHMG CoA reductase. By the term "associated" or related term is meant that the ceramide and the inhibitor are attached by at least one bond preferably at least on covalent bond. Particular examples of bonding are described below. In some instances, the association can also be provided by a suitable combination of covalent and non-covalent chemical bonds. Alternatively, association between the ceramide and the inhibitor can be provided by essential co-administration of the ceramide and the inhibitor to a desired subject mammal. More specific methods for making and using the drugs of this invention are provided in the discussion and examples which follow.

In one embodiment, the anti-lipemic drug includes the ceramide attached to the inhibitor by at least one covalent bond. As noted, preferred are recognized cholesterol inhibitors such as fluvastatin, simvastatin, lovastatin, pravastatin, mevinolin (compactin), atorvastatin. In this illustration, the ceramide is particularly any one of the preferred ceramides described previously, which ceramide is covalently linked to a reactive hydroxyl group on the inhibitor molecule. Also in this example, the hydroxyl group of the inhibitor is usually covalently linked to a reactive carbon atom on the ceramide such as the C-3 carbon.

Additional anti-lipemic drugs of this invention include at least one bifunctional spacer group, typically a heterobifunctional spacer group, which group spaces the ceramide from the inhibitor or other drug moiety. A particular example of this type of anti-lipemic drug includes one ceramide covalently linked to one heterobifunctional spacer group. That spacer group is preferably covalently linked to the serum cholesterol inhibitor. Typically, the bifunctional spacer is linked to suitably reactive chemical group on the ceramide and the inhibitor, usually specifically reactive carbon atoms and hydroxyl groups, respectively.

Preferred anti-lipernic drugs of this invention are generally formulated to suit intended use and specifically include those drugs formatted for topical or related use. Additionally, the invention includes anti-lipemic drugs that include components sufficient to provide the drug as a liposome formulation suitable for in vitro or in vivo use. Methods for making and using such preferred drugs are described below.

In general, therapeutic methods in accord with this invention include administering to a subject, particularly a mammal such as a primate, especially a human, a therapeutically effective amount of at least one anti-lipemic drug of interest. That drug can be administered as a sole active agent. Alternatively, the anti-lipemic drug can be administered in combination with other drugs or agents exhibiting a desired pharmacological activity. In most cases, the amount of anti-lipemic drug use will be one which exhibits good activity in a standard in vitro or in vivo assay described below.

As discussed, the anti-lipemic drugs of this invention advantageously provide dual "anti-cholestcrol" activity, ie, by increasing LDL receptor activity, particularly by enhancing LDL receptor levels: and by reducing serum cholesterol levels. Particular in vitro and in vivo assays to detect and quautitate these activities are provided below and in the discussion and examples which follow.

As an illustration, preferred anti-lipemic drugs of this invention are capable of stimulating production of the mature form of SREBP-1 (maturation) by at least about 2 fold, as determined by a standard SREBP-1 proteolysis (maturation) assay. That assay is provided below and generally involves monitoring in a time and dose dependent manner, the matnration of the SREBP-1 protein. Mature SREBP-1 protein is believed to move to the nucleus and stimulate production of LDL receptor.

Additionally preferred anti-lipemic drugs of this invention are capable of increasing LDL receptor mRNA levels by at least about 10%, 20%, 30%, 40%, 50%, 60%, 70%, 80, or 90% as determined by Northern blot or related mRNA detection assay. An exemplary Northern blot assay for detecting and optionally quantitating LDL receptor mRNA levels are provided below.

Also preferred anti-lipemic drugs of this invention exhibit an ID₅₀ of between from about 20%, 30%, 40%, 50%, 60%, or 70% to about 90% as determined in a standard HMG CoA reductase assay. In this assay, the activity of the reductase enzyme is monitored in the presence and absence (control) of the anti-lipemic agent. An example of the standard HMG CoA reductase assay is provided below.

Further preferred anti-lipemic drugs are capable of significantly reducing serum cholesterol as determined by a standard serum cholesterol assay. Preferably, an administered anti-lipemic drug is capable of reducing serum cholesterol in a subject mammal by at least about 5%, 10% to 20% or 30%, 40%, 50%, 60% or 70%. An example of the assay is described below. Typically, the reduction in serum cholesterol is monitored with respect to a suitable control subject. The serum cholesterol assays are optimally performed in vivo and preferably include use of a recognized animal model such as specific rabbit and mouse strains provided below.

Preferred animal models for use in the serum cholesterol assay or other suitable assay disclosed herein are generally recognized test systems for an identified cholesterol related disease. Typically such animal models include commercially available in-bred strains of rabbits or mice, e.g., the Watanabe heritable hyperlipidemic rabbit and the apolipoprotein E negative mouse. In this example, the reduction in serum cholesterol can be evaluated using well-known testing strategies adopted for use with the specific animal model. However for some applications it may be useful to test a desired anti-lipemic drug on a normal ("wild-type") animal such as those genetically defined (e.g., isogenic) wild-type animal strains known in the field.

The anti-lipemic drugs of this invention are preferably tested by at least one and preferably all of the standard assays summarized above. Preferred are anti-lipennc drugs that demonstrate about the stated activity ranges in one or more of the assays.

Significantly, use of multiple testing strategies (e.g., a combination of one in vitro and/or in vivo assays) with a single anti-lipemic drug can extend the selectivity and effectiveness of the testing as needed. That is, the testing strategy can be tailored for treatment or prevention of a particular cholesterol related disease or group of patients if required.

Such broad spectrum testing provides additional advantages. For example, preferred anti-lipemic drugs have capacity to enhance LDL receptor activity (typically by enhancing production of the LDL receptor) and provide for a reduction in scrum cholesterol level. Thus by providing such dual "anti-cholesterol" activity, the invention is a significant advance over prior therapies and agents that have been reported to reduce serum cholesterol in one way, usually by targeting cholesterol biosynthesis. Accordingly, preferred anti-lipemic drugs of this invention feature better activity, can be administered at lower dosages then prior agents. Patient tolerance of the anti-lipemic drugs will also be positively impacted.

The present invention relates to use of an anti-lipernic drug as described herein for the preparation of a pharmaceutical composition for modulating and and particularly reducing serum cholesterol level in a mammal.

Also provided is the use of anti-lipernic drug as described herein for the preparation of a pharmaceutical composition for modulating LDL receptor levels in a mammal.

The present invention also provides use of an anti-lipernic drug as described herein for the preparation of a pharmaceutical composition for treating a disorder in a mammal having or suspected of having high serum cholesterol levels. A preferred mammal is a primate and especially a human patient. e.g, those susceptible to coronary heart disease, obesity, eating disorders or other cholesterol related disorders described herein. Accordingly, the uses are especially applicable to a subject mammal such as a human patient who has been diagnosed as having, is suspected of having, or is susceptible to a high serum cholesterol level. e.g., through adverse genetic or dietary influences.

Also contemplated are methods for modulating serum cholesterol level in a mammal in which the method includes administering to the mammal a therapeutically effective amount of at least one of the anti-lipemic drugs disclosed herein. In this embodiment, the SREBP-1 effector is ceramide. Preferred methods employ a primate such as a human patient Preferred anti-lipernic agents for use in the methods are typically tested for activity using a recognized animal model for a cholesterol related disorder and especially atherosclerosis, e.g., the Watanabe heritable hyperlipidemic rabbit or an apolipoprotein E negative mouse discussed previously.

Additionally contemplated are methods for modulating LDL receptor in a mammal in which the methods include administering to the mammal a therapeutically effective amount of at least one of the anti-lipemic drugs disclosed herein. The modulation is preieiably an increase in the synthesis (or sometimes decrease in the degradation of) the LDL receptor. In this example, the SREBP-1 effector is neutral. ceramide. Methods for evaluating an increase or decrease in LDL receptor levels are known in the field and involve, e.g., molecular and immunological approaches using anti-LDL antibodies capable of detecting and quantitating LDL receptor in vitro or in vivo.

Particular uses as described above involve use use of at least one suitable anti-lipcmic drug which includes one effector of SREBP-1 associated with an identified inhibitor of serum cholesterol as discussed herein. In this example, that effector is ceramide. Preferred examples of ceramide include naturally occurring ceramide and other cefamide forms as discussed previously. As discussed, preferred methods are conducted using a mammalian subject such as a primate and especially a human patient who has been diagnosed as having, is suspected of having, or is susceptible to a cholesterol related disorder as disclosed.

In an embodiment of the disclosed herein, the anti-lipemic drug is preferably disposed as a liposome formulation. In this example, the liposome formation can be compatible for hepatic administration in accordance with standard practice. Also in this example, the liposome formulation can be administered to the liver or associated organ in a human patient according to standard medical techniques involving, e.g., oral, intramuscular, intraperitoneal, administration via a stent or related implementation. Particular routes of administration are provided below.

Other aspects of the invcution are discussed below.

### BRIEF DESCRIPTION OF THE DRAWINGS

Figure 1 is graph showing effect of TNF-α on neutral sphingomydinase (N-SMase) activity.
Figures 2A and 2B are graphs illustrating effects of TNF-α, sphinogmyelinase, and C₂ -ceramide on the kinetics of SREBP-1 maturation- 2A) kinetics of SREBP-L maturation, 2B) ratio of immature/mature SREBP-1 versus time.
Figure 2C is a representation of a Western immunoblot showing expression of TNF-α, sphingomyelinase and C₂ ceramide.
Figures 3A-C are graphs showing effects of TNF-α (3A), sphinogmyelinase (3B), and C₂ -ceramide (3C) on SREBP-1 maturation.
Figure 4 is a representation of a Western immunoblot showing effect of anti- . N-SMase amibodies on TNF-α-induced SREBP-1 maturation.
Figures 5A-D are representations of indirect immunofluorescence micrographs showing SREBP-1 expression in cells.
Figures 6A-6D are representations of gels showing results of electrophoretic mobility shift assays.
Figure 7 is a model showing how TNF-α induces SREBP-1 proteolysis(maturation) and mobilizes membrane cholesterol in human hepatocytes. Effectors of the LDL receptor and particularly SREBP-1 are shown schematically.
Figure 8 is a representation of a Western immunoblot showing N-SMase protein in cells expressing increasing amounts of a recombinant vector encoding the N-SMase (PHH1 lanes 3-6; PHHI1 lane 9).
Figure 9 is a representation of a Northern blot showing expression of the vectors encoding the N-SMase protein (lane 2 PHH1; lane 3 PHH11).
Figure 10 is a representation of a Western immunoblot illustrating SREBP-1 expression and maturation in cells.

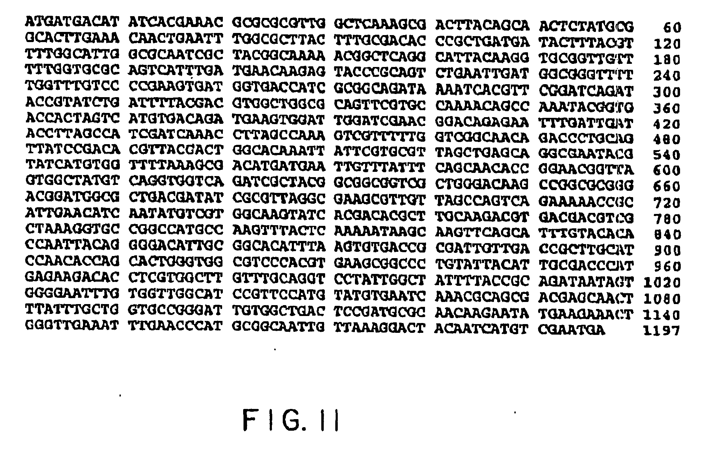

Figure 11 is a drawing showing a nucleotide sequence (SEQ ID NO:1) of isolated cDNA encoding human N-SMase.

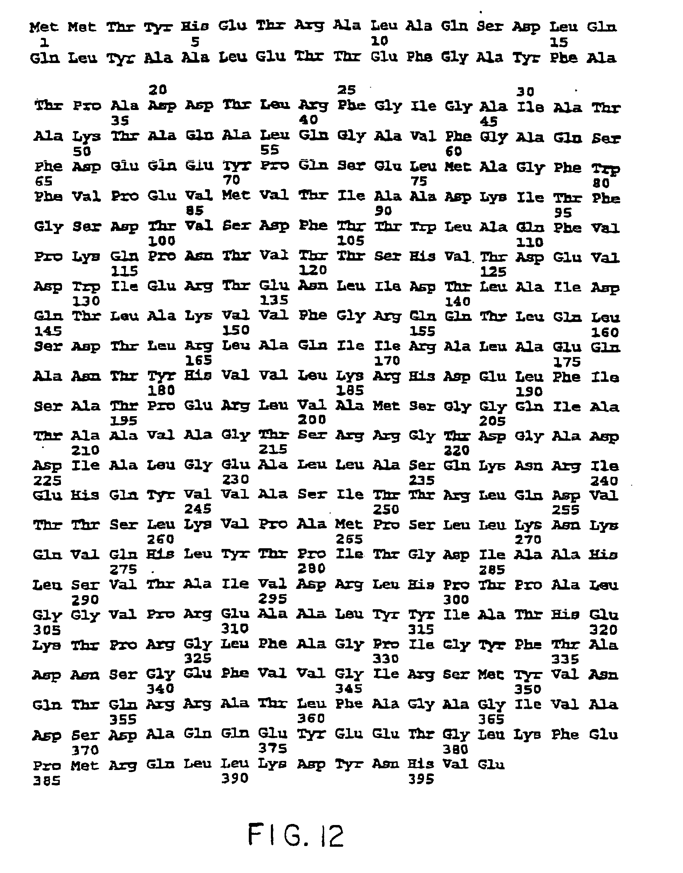

Figure 12 is a drawing illustrating the deduced amino acid sequence (SEQ ID NO:2) of human N-SMase.
Figure 13 is a drawing showing examples of particular anti-lipemic drugs, target organs and particular actions of the drugs.
Figure 14 is a drawing showing chemical structures for specific serum cholesterol inhibitors mevastatin, fluvastatin, pravastatin, lovastatin and simvastatin. The inhibitors are HMG-CoA reductase inhibitors. Fluvastatin is an entirely synthetic mevalonolactone derivative. Remaining reductase inhibitors are fungal compactin derivatives based on a hydronapthalene ring.
Figure 15A-B are drawings showing (15A) sphingomyelin and (15B) C-2 ceramide and dihydro-C-2 ceramide. The 3-hydroxyl group and 4, 5 trans carbon-carbon double bond in the sphingosine backbone are indicated by arrows.

### DETAILED DESCRIPTION OF THE INVENTION

As discussed, the invention relates to anti-lipemic drugs and uses of same to stabilize or reduce serum cholesterol level in a human patient or other subject mammal. Preferred anti-lipemic drugs generally include one identified effector of the SREBP-1 protein, a ceramide, associated with one identified serum cholesterol inhibitor. More preferred are anti-lipemic drugs in which the effector and inhibitor components are specifically covalently linked together as a single formulation.

The term "anti-lipemic drug" is used herein to refer generically to a composition of this invention, preferably a specific synthetic or semi-synthetic drug, which has dual capacity to modulate serum cholesterol levels, ie, by modulating the LDL receptor and stabilizing or reducing serum cholesterol levels in the subject mammal. Preferred is an anti-lipemic drug with demonstrated capacity to increase LDL receptor levels and to reduce serum cholesterol levels as determined by specific in vitro and in vivo assays described below. As discussed below, capacity to reduce serum cholesterol levels by the inhibitor component is generally mediated by modulation of HMG CoA reductase, typically by inhibiting that enzyme sufficient to reduce serum cholesterol. As also discussed, the effector portion preferably increases production of the LDL receptor.

The anti-lipemic drugs disclosed herein can be made by recognized methods known in the field. For example, methods for making specific ceramide and ceramide-related compounds have been disclosed in co-pending U.S. Patent Application Serial No. 08/998,262 entitled "Methods for Treatment of Conditions Associated with Lactosylceramide" filed on December 24, 1997, now issued as U.S. Patent 5,972,928 on October 26, 1999. See also Abe, A. et al., (1992) *J*. *Biochem*, 111:191-196; Inokuchi, J. et al. (1987) *J. Lipid Res*. 28:565-571; Shukla, A. et al. (1991) *J. Lipid Res*. 32:73; Vunnam, R.R. et al., (1980) *Chem, and Physics of Lipids* 26:265; Carson. K et al., (1994) *Tetrahedron Lets.* 35:2659; and Akria, A. et al.,

### (1995) J. Lipid Research 36:611.

More specific anti-lipemic drugs of this invention include as covalently linked components the effector and the serum cholesterol inhibitor, However for some applications other anti-lipemic drugs can be appropriate such as those including non-covalently linked components. Examples include those drugs provided as essentially co-administered formulations.

The molecular weight of a particular anti-lipemic drug will vary depending, e.g., on the specific ceramide and serum cholesterol inhibitor chosen and the number of cecamides and inhibitors making up the drug, However in most cases the anti-lipemic drug will have a molecular weight of less than about 10,000 kD to 35,000 kD. Molecular weights will generally be significantly lower, e.g., between from about 100 kD to 1000 kD, preferably between from about 200 kD to 500 kD. Methods for determining the molecular weight are known and include standard molecular sizing methods such as SDS polyacrylamide gel electrophoresis.

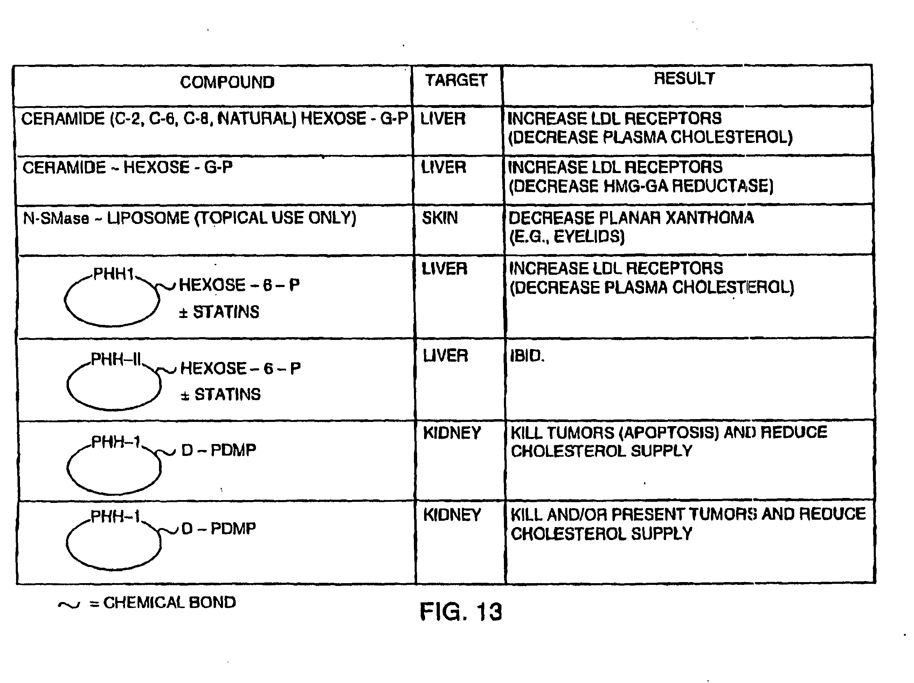

Illustrative examples of specific anti-lipemic drugs are shown in Figure 13. Figure 13 particularly shows use of combinations of SREBP-1 maturation upregulators (effectors) ceramide, N-SMase, and various lipid lowering molecules; HMG CoA-reductase inhibitors (statins) in various human pathologies.

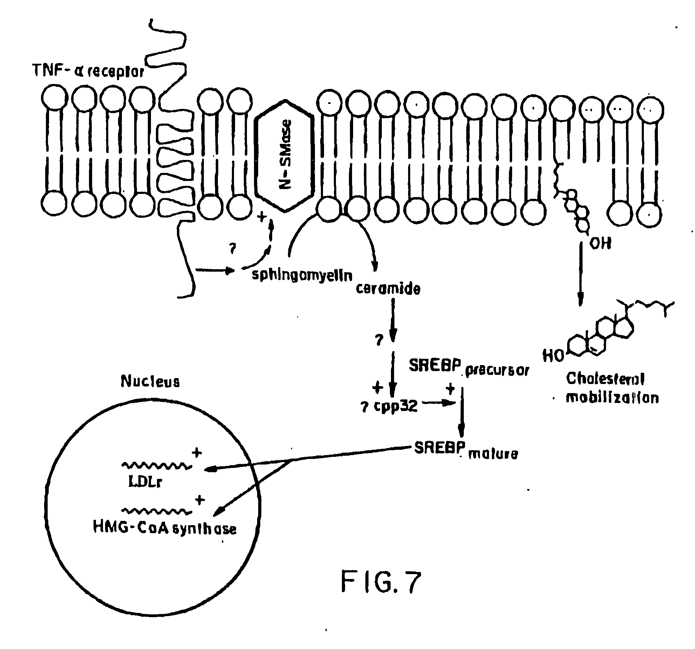

An "effector" of the LDL receptor and particularly the SREBP-1 protein is a ceramide with demonstrated capacity to modulate the LDL receptor and specifically maturation of the SREBP-1 protein as determined by the standard SREBP-1 maturation assay described below. Illustrative effectors are provided in the Examples and Figure 7.

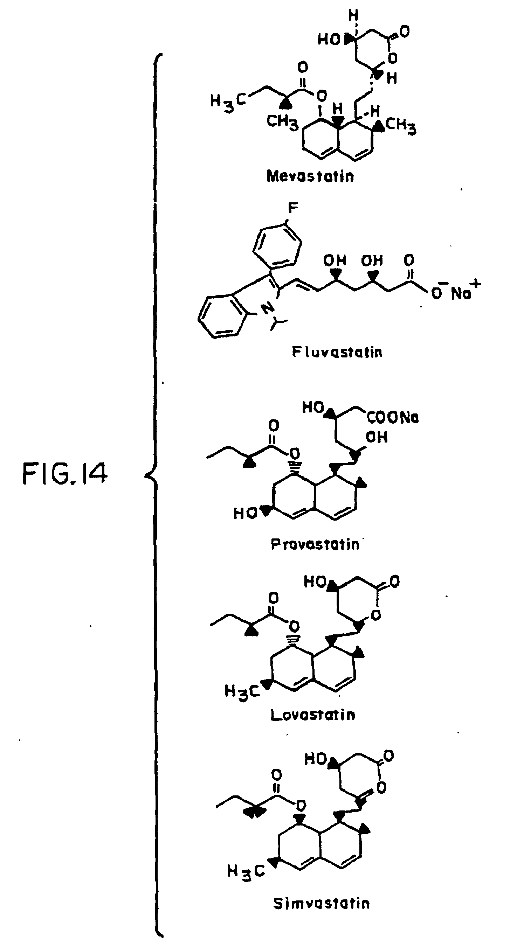

A "serum cholesterol inhibitor" as that term is used herein generally refers to a recognized compound capable of reducing serum cholesterol levels in a subject mammal and especially a human patient. Preferred serum cholesterol inhibitors are statins which interfere with cholesterol biosynthesis and especially HMG CoA-reductase activity, e.g., in the liver. More preferred serum cholesterol inhibitors are readily available commercially and include mevastatin, fluvastatin, pravastatin, lovastatin and simvastatin. See Figure 14 and the discussion below.

It has been unexpectedly found that TNF-α significantly stimulates maturation of SREBP-1 in cells through action of the N-SMase. That is, we have found that TNF-α is capable of inducing SREBP-1 maturation in a time and dose dependent manner. This induction was consistent with the kinetics of TNF-α mediated activation of neutral sphingomyelinase (N-SMase). Antibodies to N-SMase inhibited TNF-α induced SREBP-1 maturation suggesting that N-SMase is a necessary component of this signal transduction pathway. Ceramide, a product of sphingomyelin hydrolysis, was also found to be capable of inducing SREBP-1 maturation. Without wishing to be bound to theory, it appears that the mature form of SREBP-1 generated by TNF-α, sphiagomyelinase or ceramide treatment translocates to the nucleus and binds the sterol regulatory element (SRE). This is believed to promote transcription of the gene upstream of the SRE. See Figure 7 for a schematic outline of these findings. It further appears that eBectors of the SREBP-1 stimulate the LDL receptor, particularly by enhancing SREBP-1 maturation, thereby stabilizing or reducing serum cholesterol in the subject mammal.

Therapeutic methods generally comprise administration of a therapeutically effective amount of at least one and typically one anti-lipemic drug as disclosed herein to a subject mammal such as a primate and especially a human patient in such treatment. The therapeutic treatment methods more specifically include administration of an effective amount of the anti-lipemic drug to a subject, particularly a mammal such as a human in need of such treatment for an indication disclosed herein.

Typical subjects of interest include those suffering from, suspected of suffering from, or susceptible to the conditions, disorders or diseases disclosed herein, e.g., hyperlipoproteinemia including hypercholesterolemia, stroke, obesity including compulsive eating disorders, cardiac disease including atherosclerosis, cerebral atherosclerosis, cholesteryl ester storage disorder, liver disease including organ transplantation failure and cirrhosis; discases of the biliary system, and viral infection particularly those infections facilitating encephalitis or related disorders. More specific disclosure relating to these and other cholesterol related diseases including accepted methods for screening and diagnosing these disorders have been reported. See e.g., Brown. M.S. and Goldstein, J.L. (1993), *supra* and references cited therein.

A variety of specific anti-lipemic drugs can be employed in the present invention and particularly in the uses end treatment methods described. Routine testing, e.g., in a standard in vitro assay optionally combined with mother in vitro and/or in vivo assay, can in most instances readily identify suitable anti-lipemic drugs exhibiting desired selectivity and activity with respect to the target disorder or disease. As noted, preferred anti-lipemic drugs feature a specific effector of the SBREP-1 protein such as ceramide.

For example, one anti-lipemic drug of this invention includes covalently linked in sequence: 1) a ceramide comprising a chemically reactive group; and 2) a serum cholesterol inhibitor such as those disclosed herein including another chemically reactive group capable of specifically binding generally by covalent linkage to the reactive group of the effector. Optionally, the anti-lipemic drug further includes a bifunctional spacer, e.g., a heterobifunctional spacer, covalently linked between 1) and 2).

A more preferred anti-lipemic drug includes covalently linked in sequence: 1) a ceramide; and 2) a specific serum cholesterol inhibitor as disclosed herein. In this embodiment, the ceramide is preferably naturally-occurring and can be any one of C-2, C-4, C-6 or C-8 ceramide. The reactive group will typically be the C-3 group of ceramide. Preferred are serum cholesterol inhibitors that include a suitably chemically reactive hydroxyl (-OH) group, e.g., fluvastatin, simvastatin, lovastatin, pravastatin, mevinolin (comapactin), or artorvastatin. Optionally, the anti-lipemic drug may include a bifunctional spacer covalently linked between 1) and 2), ie., providing a covalent bond between the C-3 group and the hydroxyl group.

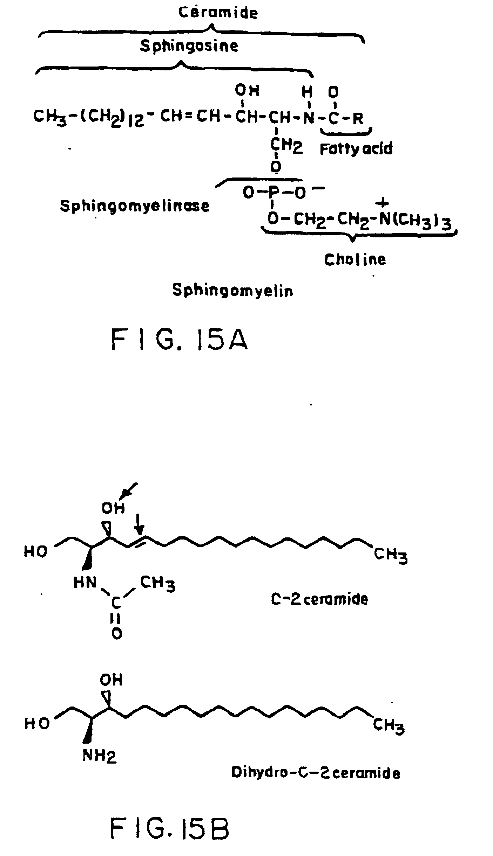

Chemical structures for sphlingomyelin and specific ceramides (C-2 ceramide, dihydro-C-2-ceramide) are shown in Figures 15A and 15B.

As discussed, preferred anti-lipemic drugs of this invention exhibit significant activity in a standard SREBP-1 maturation assay. Preferably, the drug exhibits at least about 2 fold, preferably between about 2 to 10 fold, and more preferably from about 2 to 5 fold as determined by the assay. A preferred assay generally involves:
a) culturing suitable cells, e.g, HH-25 cells, in medium and adding the anti-lipemic drug for between from about 2 to 60 minutes, preferably between from about 10 to 30 minutes with about 15 minutes being generally preferred, typically followed by washing; and
b) detecting mature SREBP-1 (ie, proteolyically cleaved) and precursor SREBP-1 by performing Western immunoblotting with an anti-SREP-1 antibody such as those described below. In general, mass of the mature form of SREBP-1 can quantitatively determined vs. the precursor form. Presence of that mature form is indicative of SREBP-1 maturation and proteolysis. More specific methods for performing the assay are provided in the Examples which follow. Typically suitable control cells are included as a reference which cells are not exposed to the drug.

As also discussed, preferred anti-lipemic drugs of this invention exhibit good activity in a Northern blot assay for detecting and preferably quantifying LDL receptor mRNA. Additionally preferred anti-lipemic drugs are capable of increasing LDL receptor mRNA levels by at least about 10% and preferably at least from between about 20% to 50% as determined by the Northern blot assay or related mRNA detection assay. Methods for performing Northern blot assays are generally known and have been described, e.g, in Sambrook et al. in *Molecular Cloning: A Laboratory Manual* (2d ed. 1989); and Ausubel et al., *Current Protocols in Molecular Biology,* John Wiley & Sons, New York, 1989.

Suitable probes for detecting LDL mRNA are generally available and include cloned sequences of the human LDL receptor or related mammalian sequence available from Genbank. Information about Genbank can be obtain from the National Library of Medicine, 38A, 8N05, Rockville Pike, Bethesda, MD 20894. Genbank is also available on the internet at http :// www. ncbi .nlm. nih.gov. See generally Benson, D.A. et al. (1997) *Nucl. Acids. Res*. 25: 1 for a more complete description of Genbank.

An exemplary Northern blot assay for detecting and optionally quantitating LDL receptor mRNA levels is discussed below.

Preferred inhibitors of the HMG CoA reductase generally reduce or block synthesis of cholesterol in the liver, thereby facilitating compensatory reactions that can lead to a reduction in plasma LDL. A preferred assay for measuring this phenomenon is the standard HMG CoA reductase assay. As mentioned previously preferred anti-lipemic drugs of this invention exhibit an ID₅₀ of between from about 20%, 30%, 40%, 50%, 60%, 70%, or 80% to about 90%, preferably between from about 30% to 50% as determined in the HMG CoA reductase assay. The standard HMG CoA reductase assay has been disclosed by Brown et al. (1978) *J. Biol. Chem*. 253:1121. In this assay cultured human fibroblasts respond to an inhibition of the reductase by accumulating increased amounts of the enzyme when compared to a suitable control.

As also discussed additionally preferred anti-lipemic drugs are capable of reducing serum cholesterol as determined by a standard cholesterol assay. The drug preferably registers at least from about 5% or 10% to 20%, 30%, 40% or 50% decrease, preferably at least about 30% to 50% decrease as determined by the assay. A preferred assay for measuring LDL cholesterol is commercially available from Sigma (St. Louis, Mo) and involves immunological separations. See also the National Cholesterol Education Program (NCEP) for information relating to acceptable cholesterol levels in humans.

A "high" or "high risk" cholesterol level or related term is defined herein as from between about 200 to 240 mg/dl (mM) cholesterol with a level greater than or equal to 240 mg/dl (mM) cholesterol being more generally understood to be indicative of high serum cholesterol. A normal serum cholesterol level is defined herein as being less than about 200 mg/dl (mM). For specific disclosure relating to conducting cholesterol tests see Brown, M.S. and Goldstein, J.L. *supra*, discussing the Guidelines of the NCEP Report of 1988.

Accordingly, "stabilization" or "reduction" of serum cholesterol as those terms are used herein will be understood to mean manifestation of a normal or near normal serum cholesterol level in the subject mammal. Also, a suitable control mammal in accord with this invention will preferably have a normal or near normal serum cholesterol level as determined by standard serum cholesterol tests.

Additional uses of an anti-lipemic drug is described herein for the preparation of a phamarceutical composition for modulating SREBP-1 levels in a mammal include administering to the mammal a therapeutically effective amount of at least one and typically one of the anti-lipemic drugs disclosed herein. Typically, modulation of the SREBP-1 is evaluated by determining maturation of the protein as determined by the SREBP-1 maturation tests described in the Examples below. A preferred assay is the SREPP-1 proteolysis assay described in the Examples.

Methods of this invention can be performed in vitro or in vivo using acceptable primary, cultured or immortalized cells such as those disclosed herein. Generally, these cells will be capable of exhibiting SREBP-1 maturation as defined herein including the HH-25 human hepatocytes described below. Methods for testing anti-lipemic drugs of interest and especially for use in human patient will preferably be conducted in vivo and may involve use of a suitable animal model depending on the method used. In this example, the model can be a suitable animal model such as those discussed previously. Alternatively, the methods can be performed on a suitable primate such as a human patient. Preferred is a human patient has been diagnosed as having, is suspected of having, or is susceptible to a cholesterol related disorder as defined above.

In embodiments in which the human patient is susceptible to one or more cholesterol related disorders, that susceptibility can be related to a genetic or environmental pre-disposition to the cholesterol related disorder. Methods for determining such pre-disposition are known in the field and include genetic testing. See Brown, M.S. and Goldstein, J.L. (1993) *supra*.

The invention thus provides uses of anti-lipemic drugs as described herein for the preparation of pharmaceutical composition for treating inappropriate (i.e. high) serum cholesterol levels as well as a disorder or condition associated therewith. Additionally comtemplated is use of the present anti-lipemic componds as prophylactic drugs to prevent development of or reduce the severity of inappropriate scrum cholesterol levels.

Compounds of the invention will be especially useful to a human patient who has or is suspected of having a cholesterol related disease, disorder or condition as defined herein. Compounds of the invention will be particularly useful in lowering serum cholesterol to normal or near normal levels in human patients. Specific examples of diseases which may be treated in accordance with the invention include hyperlipoporteinemia, stroke, cardiovascular disease and especially atherosclerosis as well as other specific disorders of conditions mentioned herein.

Without wishing to be bound by theory, it is believed the multiple and distinct covalantly linked compounds of this invention (i.e. at least one identified anti-lipemic drug in combination with at least one ceramide can significantly enhance efficacy of the anti-lipemic drug, e.g., by increasing synthesis of LDL receptor in subject cells.

Moreover, by virtue of the covalent linkage, the conjugates of the invention present the anti-lipemic drug and the ceramide to the subject cell essentially simultaneously, an effect that may not be readily achieved by administering the same compounds in a drug "cocktail" formulation without covalently linking the compounds.

It also has been reported that treatment with one drug can in turn sensitize a patient to another drug. Accordingly, the essentially simultaneous presentation to the subject cell of an anti-lipemic drug and ceramide via a conjugate of the invention may enhance drug activity, e.g., by providing synergistic results and/or by enhancing production of LDL receptors.

Administration of compounds of the invention may be made by a variety of suitable routes including oral, topical (including transdermal, buccal or sublingal), nasal and parenteral (including intraperitoneal, subcutaneous, intravenous, intradermal or intramuscular injection) with oral or parenteral being generally preferred. It also will be appreciated that the preferred method of administration and dosage amount may vary with, for example, the condition and age of the recipient.

Compounds of the invention may be used in therapy in conjunction with other medicaments such those with recognized pharmacological activity to lower concentrations of plasma lipoproteins. See Brown, M.S. and Goldstein, J.L. *supra*. Exemplary medicaments are recognized serum cholesterol inhibitors (i.e. reported to inhibit HMG CoA reductase) such as Lescol™ (fluvastatin from Sandoz Pharmaccuticals), Mevacor™ and Zocor™ (simvastatin and lovastatin, respectively, from Merck & Co.), Pravachol™ (pravastatin from Bristol-Myers Squibb Co.) and mevinolin (compactin).

The compounds of this invention may be used alone or in combination with other accepted anti-lipemic therapies including those implementing use of fibric acids, e.g., gembibrozil, clofibrate, fenofibrate, ciprofibrate or bezafibrate; bile acid-binding resins such as cholestyramine or colestipol; and probucol. The compounds of this invention can be administered before, during or after such therapies as needed.

While one or more compounds of the invention may be administered alone, they also may be present as part of a pharmaceutical composition in mixture with conventional excipient, i.e., pharmaceutically acceptable organic or inorganic carrier substances suitable for parenteral, oral or other desired administration and which do not deleteriously react with the active compounds and are not deleterious to the recipient thereof. Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

For parenteral application, particularly suitable are solutions, preferably oily or aqueous solutions as well as suspensions, emulsions, or implants, including suppositories. Ampules are convenient unit dosages.

For enteral application, particularly suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc.

Therapeutic compounds of the invention also may be incorporated into liposomes. The incorporation can be canied out according to known liposome preparation procedures, e.g. sonication and extrusion. Suitable conventional methods of liposome preparation are also disclosed in e.g. A.D. Bangham et al., *J. Mol. Biol*., 23:238-252 (1965); F. Olson et al., *Biochim. Biophys. Acta,* 557:9-23 (1979); F. Szoka et al., *Proc. Nat. Acad. Sci*., 75:4194-4198 (1978); S. Kim et al., *Biochim. Biophys. Acta,* 729:339-348 (1983); and Mayer et al., *Biochim. Biophys. Acta,* 858:161-168 (1986).

The liposome may be made from one or more of the conjugates discussed above alone, or more preferably, in combination with any of the conventional synthetic or natural phospholipid liposome materials including phospholipids from natural sources such as egg, plant or animal sources such as phosphatidylcholine, phosphatidylethanolamine, phosphatidylglycerol, sphingomyelin, phosphatidylserme or phosphatidylinositol. Synthetic phospholipids also may be used e.g., dimyristoylphosphatidylcholine, dioleoylphosphatidylcholine, dioleoylphosphatidycholine and corresponding synthetic phosphatidylethanolamines and phosphatidylglycerols. Cholesterol or other sterols, cholesterol hemisuccinate, glycolipids, 1,2-bis(oleoyloxy)-3-(trimethyl ammonio)propane (DOTAP), N-[1-(2,3-dioleoyl)propyl]-N,N,N-trimethylammonium chloride (DOTMA), and other cationic lipids may be incorporated into the liposomes. The relative amounts of the one or more compounds and additives used in the liposomes may vary relatively widely. Liposomes of the invention suitably contain about 60 to 90 mole percent of natural or synthetic phospholipid; cholesterol, cholesterol hemisuccinate, fatty acids or cationic lipids may be used in amounts ranging from 0 to 50 mole percent; and the one or more therapeutic compounds of the invention may be suitably present in amounts of from about 0.01 to about 50 mole percent.

It will be appreciated that the actual preferred amounts of active compounds used in a given therapy will vary according to the specific compound being utilized, the particular compositions formulated, the mode of application, the particular site of administration, etc. Optimal administration rates for a given protocol of administration can be readily ascertained by those skilled in the art using conventional dosage determination tests conducted with regard to the foregoing guidelines.

In general, for treatment of a lipid related disease as disclosed herein and particularly hyperlipoproteinemia, stroke, coronary heart disease and especially atherosclerosis, a suitable effective dose of one or more compounds of this invention will be in the range of from 0.01 to 100 milligrams per kilogram of bodyweight of recipient per day, preferably in the range of from 0.1 to 50 milligrams per kilogram bodyweight of recipient per day, more preferably in the range of 1 to 20 milligrams per kilogram bodyweight of recipient per day. The desired dose is suitably administered once daily, or several sub-doses, e.g. 2 to 5 sub-doses, are administered at appropriate intervals through the day, or other appropriate schedule.

A preferred dose for many compounds of this invention will be in the range of those dosages accepted for identified HMG CoA reductase inhibitors with lower than that range being preferred for many patients.. See the *Physicians' Desk Reference, supra* for more specific information relating to recommended doses for HMG CoA reductase inhibitors with anti-lipemic activity.

The present invention also provides methods for determining therapeutic capacity of the anti-lipemic drug disclosed herein for treating a cholesterol related disease in a mammal. In one embodiment, the method includes:
a) providing a population of cells capable of expressing SREBP-1,
b) contacting the cells with the anti-lipemic drug in an amount sufficient to induce maturation of the SREHP-1,
c) culturing the cells in medium; and
d) detecting maturation of the SREBP-1 as indicative of the therapeutic capacity of the anti-lipemic drug in treating the disease.

Also provided herein are methods for determining therapeutic capacity of one or more of the anti-lipemic drugs disclosed herein using a Watanabe heritable hyperlipidemic rabbit or spolipoprotein and negative mouse as an animal model. In one embodiment, the method includes:
a) administering at least one of the anti-lipemic drugs to the rabbit or mouse in an amount sufficient to reduce serum cholesterol levels by at least from about 10 to 20% as determined by a standard cholesterol assay; and
b) detecting the serum cholesterol reduction in the rabbit or mouse as being indicative of the therapeutic capacity of the anti-lipemic drug to treat the cholesterol related disease.

Methods of this invention can optionally include monitoring LDL receptor activity as being indicative of the effector of the SREBP-1. In this embodiment, the receptor activity can be suitably monitored and quantified if desired by one or a combination of standard strategies. For example, a variety of specific methods have been reported to monitor LDL receptor activity and particularly to detect increases or decreases in the level of LDL receptors. See Brown, M.S. and Goldstein, J.L. (1993), *supra* and references cited therein for several immunological and molecular approaches. A preferred method is the standard LDL receptor Northern blot assay disclosed herein.

Suitable cells for use in the methods of this invention are described in the Examples which follow.

Preferred are cells which include SREBP-1 and are capable of SREBP-1 maturation as determined by the standard assay described herein. More prefarud are cells responsive to an increase or decrease in ceramide or a related compound such as human hepatocytes as provided in the Examples below.

It is preferred that the anti-lipemic drugs as well as components thereof (e.g., ceramide) be substantially pure. That is, the drugs will be present in at least 90 to 95% homogeneity (w/w). Anti-lipemic drugs having at least 98 to 99% homogeneity (w/w) are most preferred for many pharmaceutical, clinical and research applications. Once substantially purified the drug should be substantially free of contaminants for therapeutic applications. Once purified partially or to substantial purity, the drugs can be used therapeutically, or in performing preferred *in vitro* or *in vivo* assays as disclosed herein- Substantial purity can be determined by a variety of standard techniques such as chromatography and gel electrophoresis.

The Examples 1-8 below illustrate that TNF-α is capable of inducing SREBP-1 proteolysis independent of the presence of sterols. Exogenously supplied sphingomyelinase and ceramidc are also capable of inducing SREBP-1 proteolysis in a time and dose dependent manner. The kinetics of SREBP-1 maturation is consistent with those of neutral sphingomyelinase activation by TNF-α. Further, SREBP-1 maturation can be blocked with anti-N-SMase antibodies indicating that neutral sphingomyelinase is necessary for TNF-α induced, sterol independent SREBP-1 cleavage. The product of sterol independent SREBP-1 proteolysis is capable of nuclear translocation and binds to the sterol regulatory element.

The following abbreviations are used throughout this disclosure including the following examples as needed: N-SMase, neutral sphingomyelinase; LDLr, Low Density Lipoprotein receptor, SREBP-1, Sterol Regulatory Element Binding Protein-1. Numbered citations are listed in numerical order below.

### Example 1- The effect of TNF-α on Neutral sphingomyelinase activity

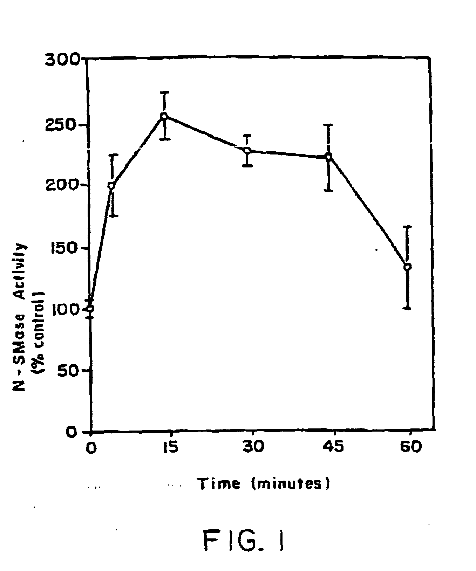

Neutral sphingomyelinase activity increased rapidly with the addition of TNF-α. See Figure 1. A maximal 2.5 fold increase in activity was observed 15 minutes after TNF-α was added to the cells. The gradual retum of N-SMase activity to control levels within 1 hour contrasted the rapid onset of activation and is reflected in the asymmetric kinetic profile observed.

Figure 1 illustrates the effect of TNF-α on neutral sphingomyclinase activity and is explained in more detail as follows: Confluent cultures of HH-25 cells were washed once with PBS and incubated in serum free media for 30 minutes prior to the addition of TNF-α (10ng/ml). At the indicated time, cells were harvested in PBS, pelleted and frozen. Cells were subsequently lysed as described in materials and methods. N-SMase assays were performed in duplicate as described. Error bars represent ± one standard deviation from the mean.

### Example 2 Kinetics of SREBP-1 proteolysis

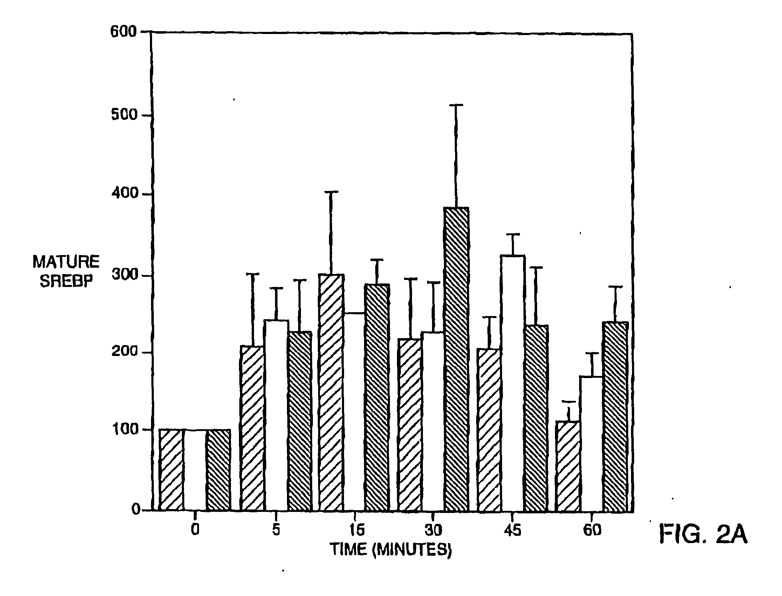

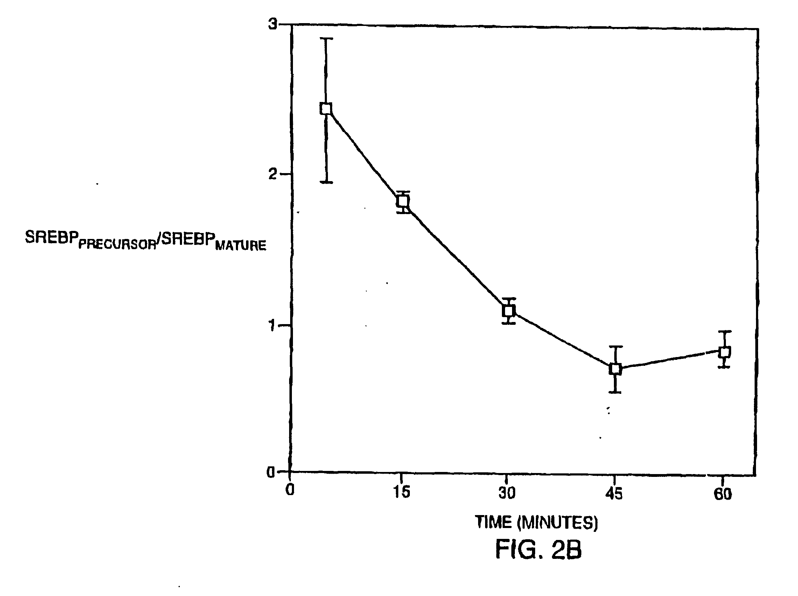

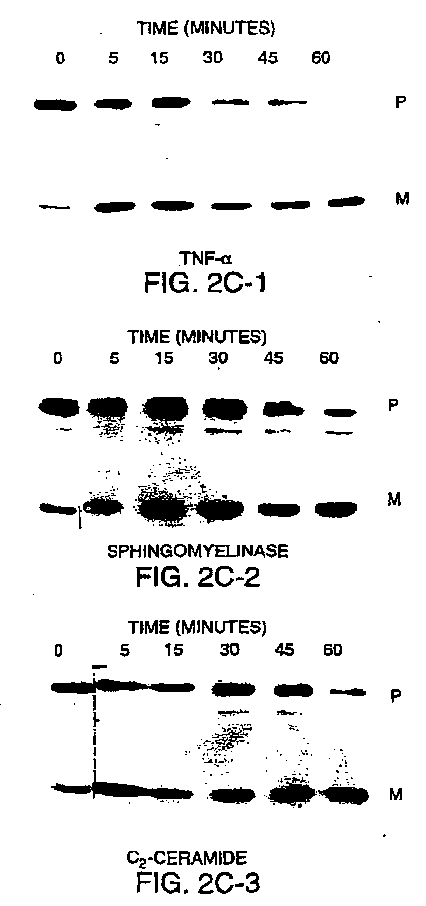

Sterol independent SREBP-1 maturation in response to TNF-α closely paralleled the kinetics of INF-α induced N-SMase activation. The mass of the mature form of SREBP-1 was found to increase 2 fold after 5 minutes and 3 fold after 15 minutes of incubation with TNF-α. See Figure 2A. The amount of mature SREBP-1 returned to control levels within one hour. This effect could not be recapitulated with BGF or PDGF treatment. The increase in mature SREBP-1 levels was accompanied by a concomitant decrease in the intensity of the band corresponding to the precursor form of SREBP-1. See Figure 2B. After 60 minutes of treatment significantly less precursor SREBP-1 was visible.

To incorporate the observed increase in mature SREBP-1 and the concomitant decrease in precursor SREBP-1 into a single variable, the ratio of precursor SREBP-1 to mature was plotted. See Figure 2B. A maximal 1.5 fold decrease in the precursor to mature ratio occurred 45 minutes after TNF-α was added to the media. The decrease in precursor to mature ratio was more pronounced in the initial 30 minutes of treatment. This is also consistent with the kinetics of TNF-α induced N-SMase activation.

To explore the possibility that plasma membrane sphingomyelinase was involved in the signal transduction pathway leading to SREBP-1 proteolysis, cells were treated with exogenously supplied bacterial sphingomyelinase. Sphingomyelinase induced a dramatic change in the relative amounts of precursor and mature SREBP-1. As seen in Figures 2A-2B, a 2.5 fold increase in mature SREBP-1 levels was observed after 15 minutes treatment. Unlike TNF-α, the increase in mature SREBP-1 induced by sphingomyelinase persisted after 60 minutes. Sphingomyelinase was also capable of reducing the level of the precursor form of SREBP-1. See Figures 2A-B. Treatment with purified recombinant human sphingomyelinase produced similar results.

Much of the signal transducing ability of N-SMase has been asenbed to its ability to genemte the lipid second messenger ceramide. Accordingly, the ability of a cell permeable caramide analog C₂-ceramide (N-acetylsphingosine) was tested to induce SREBP-1 maturation. C₂-ceramide also induces SREBP-1 maturation in a sterol independent manner with greater magnitude than what was observed with either TNF-α or sphingomyelinase. C₂-ceramide increased the level of mature SREBP-14 fold after 30 minutes of treatment . See Figures 2A-2B. The persistent elevation of mature SREBP-1 levels observed with sphingomyelinsse treatment also accompanied C₂-ceramide treatment. The increase in mature SREBP-1 is recapitulated with the addition of bovine brain ceramides but could not be induced with C₂₋dihydrocaamide, PL-A₂, or Phospholipid D treatment .

The kinetics of SREBP-1 maturation presented in this example would suggest that SREBP-1 proteolysis is a sufficiently early event to be involved in providing cholesterol to apoptotic cells. However, there was no evidence of apoptosis in the HH-25 human hepatocyte cell line used in this study. Without wishing to be bound to theory, it is conceivable that the sterol independent induction of SREBP-1 maturation in hepatocytes is a physiologic process that does not require that apoptosis be induced. Alternatively, the two pathways may diverge before the cell has been committed to apoptosis suggesting a manner in which sterol independent SREBP-1 proteolysis could be employed independent of the induction of apoptosis.

The sterol-independent cleavage of SREBP-1 observed with human hepatocytes could also occur by ceramide generated by the TNF-α induced N-SMase activation. This phenomenon may be reconstituted by the exogenous addition of N-SMase and/or C₂ ceramide to the hepatocytes.

Figures 2A-2C illustrate effects of TNF-α sphingomyelinase and C₂-ceramide on the kinetics of SREBP-1 maturation- Figures 2A-2C is explained in more detail as follows: Cells were maintained in media supplemented with 1 µg/ml 25-hydroxy cholesterol and 15 µg/ml cholesterol for 24 hours before the experiment The cells were treated for the indicated time as described in materials and methods. The cells were then harvested in PBS. pelleted and frozen. Lysis and nuclear fractionation were performed as described. Nuclear fiuctions (50µg of protein) were electrophoresed on a 7.5% polyacrylamide gel and transferred to a PVDF membrane. Western blotting was performed as described. Band intensity was quantised via densitometry. Error bars represent ± one standard deviation from the mean. 2A) The kinetics of SREBP maturation as measured by the increase in mature SREBP-1 are plotted. Fold increase was calculated by comparing each time point to the corresponding control value (TNF-α is represented by stippled bars, bacterial sphingomyelinase is represented by light gray bars and C₂-ceramide by the dark bars.) 2B) Cells were treated with TNF-α (10ng/ml) and prepared as described above. The bands corresponding to the precursor and mature forms of SREBP-1 were quantified and their ratio plotted. 2C) Representative Western blots from which numerical data was derived. Incubation time is indicated above and applies to all conditions. The membranes were exposed to film for 15 seconds. P and M denote the precursor and mature forms of SREBP-1 respectively.

### Example 3- Effects of INF-α, sphingomyetmase and C₂-ceramide on apoptosis in hepatocytes

To demonstrate that the observed maturation of SREBP-1 was not an artifact of the more general phenomenon of apoptosis induced proteolysis we performed DNA laddering assays. The 160 bp DNA ladder characteristic of cells undergoing apoptosis was not observed in any of the samples.

TNF-α, C₂-ceramide and sphingomyelinase did not induce apoptosis demonstrating that in hepatocytes, SPXBP-1 maturation is not part of the more general phenomenon of apoptotic protein hydrolysis.

### Example 4- Effects of TNF-α, sphingomyelinase and C₂-ceramide concentration on SREBP-1 maturation

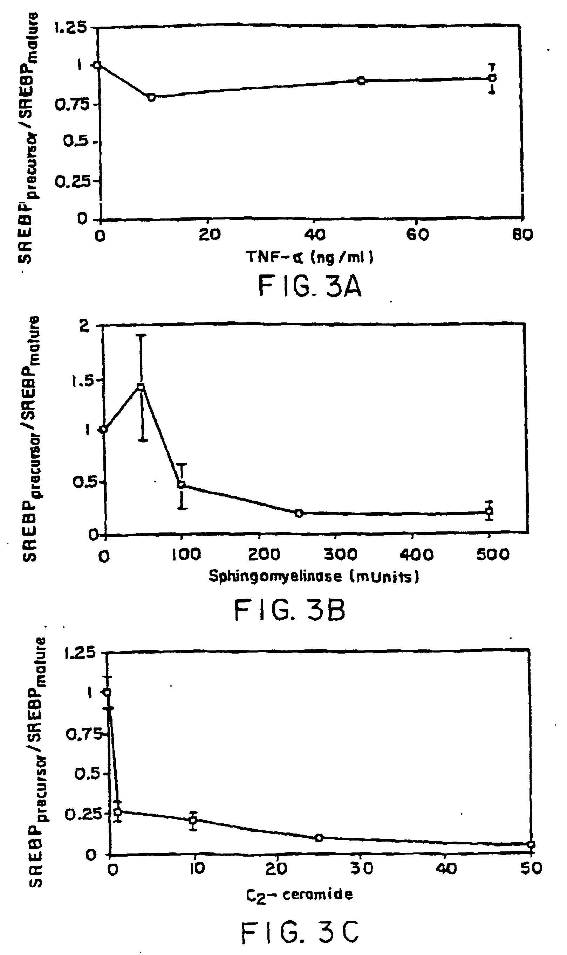

The extent of TNF-α induced SREBP-1 manuation did not vary appreciably with concentration. A maximal effect was observed with 10ng/ml of TNF-α. See Figures 3A-C. 250 milliunits of sphingomyelinase activity induced an 80% decrease in the precursor to mature ratio. As little as 1µM of C₂-ceramide was effective in producing an 81% maximal effect. The maximal effect however, was obtained with a C₂-ceramide concentration of 50 M. See Figures 3A-C.

Figures 3A-3C show effects of TNF-α, sphingomyelinase and C₂-ceramide concentration on SREBP-1 maturation- The Figures 3A-3C are explained in more detail as follows. Cells were treated with either TNF-α, sphingomyelinase or C₂₋ceramide at the indicated concentrations. Nuclear pellets were prepared and electrophoresed (50µg of protein). The bands corresponding to the precursor and mature froms of SREBP-1 were quantified. The precursor to mature ratios were normalized to a single control to facilitate comparison. The control ratio was arbitrarily assigned a value of 1. A Unit of sphingomyelinase activity hydrolyzes 1.0 µmol of spbingomyclin per minute at 37°C. Figure 3A(ng/ml TNF-α); Figure 3B (mUnits of sphingomyelinase); Figure 3C (micromolar C2-ceramide)

### Example 5- The effect of anti-N-SMase antibodies on TNF-α mediated SREBP-1 manuration

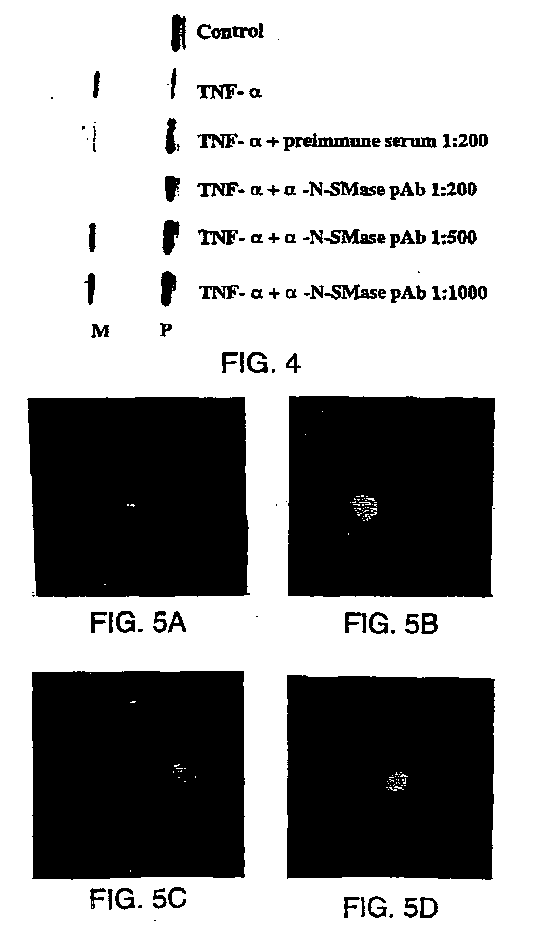

The availability ofanti-N-SMase antibodies allowed us to examine the effects of TNF-α on this pathway independent of N-SMase activation (10). Polyclonal anti-N-SMase antibodies at a dilution of 1:200 completely block TNF-α induced SREBP-1 maturation. See Figure 4. The suppression of TNF-α mediated SREBP-1 matnration was relieved with increasing antibody dilution. Preincubation with preimmune serum at the same dilution bad no appreciable effect.

This example shows that pre incubation with anti-N-SMase antibody effectively blocked TNF-α induced SREBP-1 maturation. Inhibition was not observed with pre-immune serum treatment and was relieved with increasing antibody dilution. Such findings are confirmed by other studies such as those showing the ability of the antibody to inhibit TNF-α induced increases in cholesterol ester synthesis and N-SMase induced increases in ^{[125]}I-LDL binding, internalization and degradation in human fibroblasts (15, 16).

Figure 4 shows effect of anti-N-SMase antibodies on TNF-α induced SREBP-1 maturation. The Figure 4 is explained in more detail as follows. Cells were maintained in media supplemented with 1 µg/ml 25-hydroxycholesterol and 15 g/ml cholesterol for 24 hours before the experiment. The cells were switched to serum free media for 15 minutes and then incubated with anti-N-SMase antibodies or rabbit preimmune serum at the indicated dilution for 30 minutes prior to TNF-α addition (10ng/ml). The cells were then harvested, pelleted and lysed as described. The samples were electrophoresed on a 7.5% polyacrylamide gel and transferred to a PVDF membrane. Bands were visualized as described. Film was exposed for 15 seconds.

### Example 6- Effects of TNF-α, C₂-ceramide and Sphingomyelinase on the subcellular localization of SREBP-1

To determine if the SREBP-1 fragment generated by TNF-α, C₂-ceramide or sphingomyelinase treatment was capable of nuclear translocation, immunofluorescence studies were persued. Previous immunofluorescence studies have relied on the overexpression of precursor and mature forms of SREBP-1 (14). We were able to visualize endogenous SREBP-1 in treated and untreated cells with polyclonal antibodies directed against the DNA binding domain of SREBP-1. Since the DNA binding domain is common to both the precursor and mature forms, examination of the total distribution of endogenous SREBP-1.was possible.

TNF-α, C₂-ceramide and sphingomyelinase all are capable of inducing changes in the subcellular localization of SREBP-1. See Figure 5A. Untreated cells display an even staining pattern throughout their cell bodies. This is consistent with the localization of precursor SREBP-1 to intracellular membranes (14). However, cells treated with TNF-α, C₂-ceramide or sphingomyelinase all exhibit intense nuclear staining and little extra-nuclear staining. See Figures 5B-5D. The rapid change in the subcellular localization of SREBP-1 is consistent with a precursor/product relationship between the two forms and provides additional evidence that the mature SREBP-1 fragment generated by treatment is capable of nuclear translocation.

Figures 5A-5D show indirect immunofluorescence of SREBP-1. Figures 5A-5D are discussed in more detail as follows. SREBP-1 was visualized with rabbit polyclonal antibodies directed towards the N-terminal DNA binding domain which is common to both the precursor and mature forms. Cells were maintained in media supplemented with 1µg/ml 25-hydroxycholesterol and 15 µg/ml cholesterol for 24 hours before the experiment. Immunofluorescence was performed as described. All magnifications are 40X and all photographs were taken of samples that were fixed 30 minutes after initiating treatment. Figure 5A) Control cells, Figure 5B) Cells treated with TNF-α (10ng/ml), Figure 5C) Cells treated with sphingomyelinase (100mUnits), Figure 5D) Cells treated with C₂-ceramide (10µM).

### Example 7-Electrophoretic Mobility Shift Assays

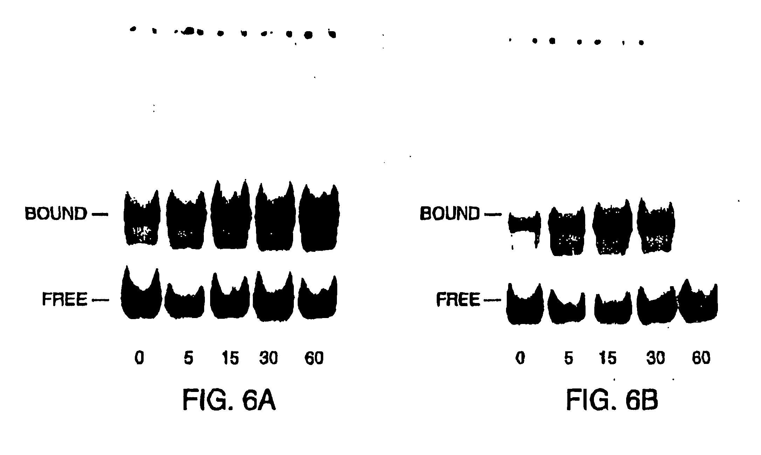

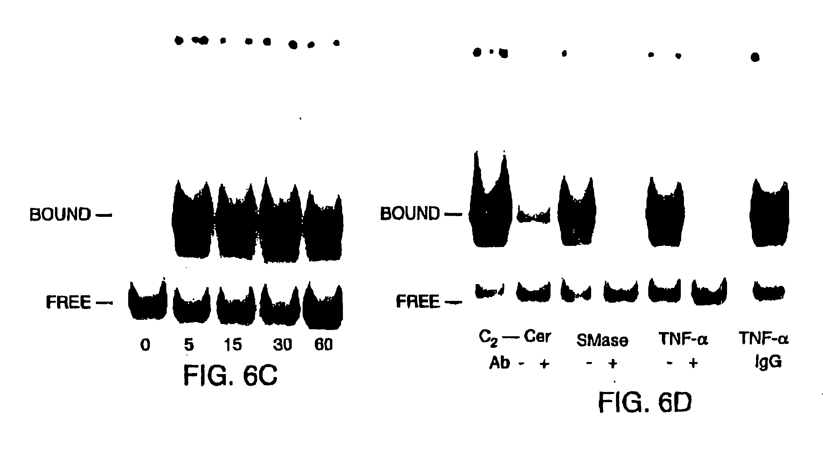

Electrophoretic mobility shift assays were pursued to demonstrate that the mature SREBP-1 fragment is additionally capable of binding to its consensus sequence. The amount of electorphoretically retarded probe increases with time following TNF-α treatment. See Figure 6A. The kinetics of this process is consistent with the activation of N-SMase. The amount of probe bound increases with sphingomyelinase and cstamide treatment. As expected, C₂-ceramide induces a more rapid accumulation of active, nuclear SREBP-1 than either TNF-α. or sphingomyelinase. See Figures 6A-6C. Antibodies directed towards the DNA binding domain of SREBP successfully compete with the oligonucleotide probe for binding. See: Figure 6D. Binding of the probe is not titrated by an unrelated oligonucleotide but is decreased with the addition of a non-radioactive competing probe.

Figures 6A-6D show electrophoretic mobility shift assays. Figures 6A-D are explained in more detail as follows. Cells were maintained in sterol supplemented media. Nuclear pellets were prepared and assayed as described in materials and methods. Probe that has been bound by mature SREBP-1 is indicated as "Bound". Unbound probe is indicated as "Free". The kinetics (in minutes) of SREBP-1 binding to the probe in response to treatment with (Figure 6A). TNF-α (10ng/ml), (Figure 6B) sphingomyelinase (100mUnits) and (Figure 6C) C₂-ceramide (10 M). (Figure 6D). The ccus were treated with either TNF-α (10ng/ml), sphingomyelinase (100mUnits) or Cz-ceramide (10µM) for 15 minutes. Sopeashift assays were then performed with antibodies raiscd against the DNA binding domain of SREBP-1. The presence or absence of antibody is indicated by (+) and (-) respectively. Pre-immune IgG was used as a control.

The gel mobility shift experiments in Figures 6A-D clearly indicate that TNF-α, N-SMase and C₂ ceramide all induce SREBP-1 levels in hepatocytes. It is known that TNF-α induces sterol metabolism in cultured human fibroblasts (15) and LDL receptors (16,17). The present data indicate that indeed TNF-α induces LDL receptor mRNA level in human hepatocytes. One result is that TNF-α induced increase in mature SREBP-1 level is accompanied by increased LDL receptors and sterol metabolism.

**Example 8**- Effects of Overexpression of Neutral sphingomyelinase (N-SMase) and Recombinant N-SMase on the Maturation of Sterol-regulatory element binding protein-1 and Low density Lipoprotein Receptor Expression in cultured Human Hepatocytes

The present example was conducted to address whether the overexpression of N-SMase employing two separate N-SMase plasmid DNA(PHH-1,representing the entire uncleotide sequence in N-SMase cDNA and PHH-11, representing nucleotide sequence 862-1414) would increase the maturation of SREBP-1 and LDL receptor mRNA expression in a human hepatocyte cell line HH-11. Cells transfected with mock plasmid cDNA(PSV-SPOT) served as a control and cells incubated with C-2 ceramide previously shown to induce SREBP-1 maturation served as a positive control.

Briefly, human hepatocytes(1x10⁴) were seeded in sterile 100 mm² in medium containing 10% dialyzed, heat inactivated fetal bovine serum without antibiotics. Twenty four hours later medium was replaced with 9 ml of fresh serum free medium. After incubation for 30 min at 37°C 5-40 µg of the plasmid DNA in 1 ml of a CaCl₂ solution (mixed with equal volume of 0.25-2.5 M CaCl₂ solution in HEPES buffer and HEPES buffer pH 6.95). Following gentle mixing incubation of cells was continued for 5-24 hr at 37 °C. The transfection reaction was terminated by removing the medium and washing the cells with scrum free medium. Next, fresh serum supplemented medium was added and incubation was continued for an additional 24 hr and cells were harvested in appropriate buffer centniuged and stored frozen until further analysis. Cell pellets were homogenized and suitable aliquots subjected to Western immunoblot analysis as described below and in Examples 1-7 above. Total RNA was isolated from another batch of cells transfected as above and subjected to Northern analysis employing a ³²P labeled LDL receptor consensus sequence. The autoradiographs were developed and photographed.

Cells transfected with 0.2µg/ ml of PHH1 or FHH11 showed a 2-fold increase in N-SMase activity compared to mock cDNA transfected cells. This was accompanied with aPHHland PHH11 concentration dependent increase in the maturation of SREBP-1 in human hepatocytes. See Figure 7. As shown in lanes 3-6 transfection of cells with 5,10,20,40 µg of PHH1 plasmid DNA/dish resulted in a gradual but marked increase in the maturation of SREBP-1 as compared to mock cDNA transfected cells (lane1,Figure 7). In contrast, a marked increase in the maturation of SREBP-1 was noted in cells transfected with 20µg/dish of PHH11 plasmid DNA (lane 9 Figure 7) but subsided at a higher concentration. As expected form the Examples 1-7 above, cells incubated with C-2 ceramide (µM) markedly increased the maturation of SREBP-1(lane 2 Figure 7). In additional experiments we observed that increasing the time of transfection from 8 hr to 24 hr decreased the maturation of SREBP-1 in hepatocytes. Moreover, decreasing the concentration of CaCl₂ from 2.5M to 0.25M was ineffective.

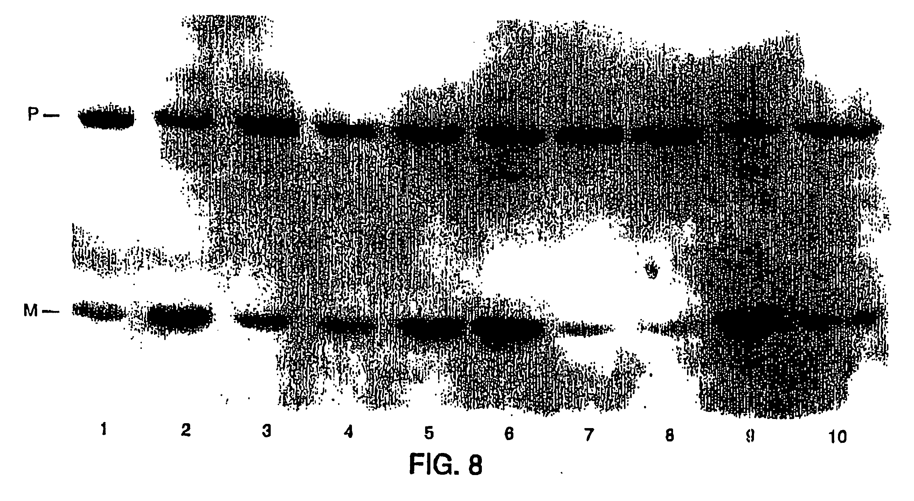

Northern gel analysis revealed that transfection with PHH1 and PHH11(lanes 2, 3, respectively in Figure 8) significantly increased the level of LDL receptor mRNA as compared to cells transfected with mock cDNA (lane 1 Figure 8).

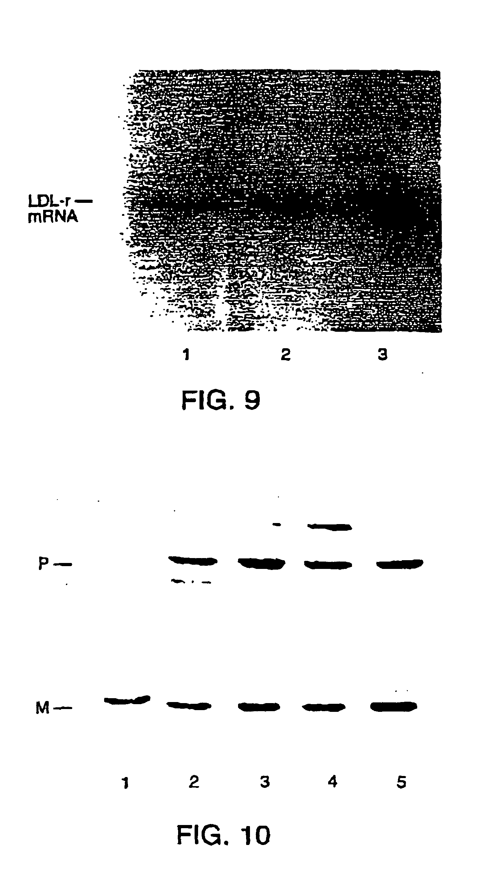

In another experiment hcpatocytes were incubated with purified bacterial recombinant N-SMase. Preferred methods of making and using the recombinant N-SMase are described in the co-pending U.S. Patent Application Serial No.08/774,104, now issued as U.S. Patent 5,919,687. That N-SMase was subjected to western immunoblot analysis employing antibody against SREBP-1. As shown in Figure 9, cells incubated with C-2 ceramide markedly increased the maturation of SREBP-1 (lane1). In comparison the r-N-SMase exerted a concentration -dependent increase in the maturation of SREBP-1 (lane 2, 3, 4, 5 representing 0.4, 0.8, 2,and 4 µg/ml of r-N-SMase, respectively).

This example shows that overexpression of N-SMase or feeding r-N-SMase to hepatocytes stimulates the maturation of SREBP-1 and consequently an increase in the LDL receptor mRNA levels.

The Examples 1-8 above highlight a novel pathway by which SREBP-1 maturation could be effected in a sterol independent manner. It was found that TNF-α is capable of inducing SREBP-1 maturation in a sterol independent manner in human hepatocytes. These findings are not a general response to growth factors, as they could not be recapitulated with EGF or PDGF. The maturation, nuclear translocation, and SRE binding activity of SREBP-1 in response to TNF-α closely paralleled the kinetics of N-SMase activation. The effect of TNF-α on SREBP-1 maturation could be reconstituted with exogenously supplied bacterial or human sphingomyelinase C₂₋ceramide but could not be recapitulated with dihydroceramide, PL-A₂, or PL-D.

In particular. Examples 1-7 show that addition of C₂-ceramide, a water soluble ceramide analog, or bacterial sphingomyelinase mimicked the effect of TNF-α on SREBP-1 maturation. C₂-ceramide and sphingomyelinase induced more extensive SREBP-1 maturation than TNF-α. Without wishing to be bound to theory, this observation may reflect the presence of a regulatory event upstream of ceramide generation that is effectively bypassed with exogenous ceramide or sphingomyelinase. Also, the lack of apparent dose dependence observed with TNF-α treatment might be attributable to saturable binding of the TNF-α receptors or an internal regulatory event that reduces the signaling capacity of the TNF-α receptors.

The present data and discussion indicate a model in which TNF-α initiates SREBP-1 proteolysis. The model (Figure 7) in which there is shown TNF-α binding to one or more of its cell surface receptors and in so doing promotes the activation of N-SMase. N-SMase hydrolyzes membrane sphingomyelin into ceramide and phosphocholine. Ceramide, in turn, activates a protease perhaps CPP32 that mediates SREBP-1 maturation. According to the model, the mature SREBP-1 then migrates into the nucleus as shown and drives the transcription of genes with an upstream sterol regulatory element.

The model illustrated in Figure 7 clarifies how sterol homeostasis can occur in the presence of increased cytosolic sterols, which would be predicted to suppress SREBP-1 maturation. One advantage conferred by the participation of neutral sphingomyelinase in cholesterol homeostasis is that it is capable of providing a short term solution to cholesterol starvation through mobilization of plasma membrane cholesterol and can facilitate long term compensatory mechanisms by promoting the maturation of SREBP-1.

The model shown in Figure 7 also shows that TNF-α is capable of inducing SREBP-1 proteolysis independent of the presence of sterols. Exogenously supplied sphingomyelinase and ceramide are also capable of inducing SREBP-1 proteolysis in a time and dose dependent manner. The kinetics of SREBP-1 maturation is consistent with the activation of neutral sphingomyelinase by TNF-α. Furthermore, recombinant human N-SMase can also exert a time and concentration dependent induction of SREBP-1 maturation. In addition, anti-N-SMase antibodies block SREBP-1 maturation. These findings indicate that neutral sphingomyelinase is necesmy for TNF-α induced, sterol independent SREBP-1 cleavage.

The present examples and discussion identify N-SMase in the TNF-α initiated signal transduction pathway leading to SREBP-1 maturation amd provide evidence that ceramide is the second messenger employed. Also shown is an important role for TNF-α in the regulation of cholesterol homeostasis.

The present findings are summarized as follows. The role of TNF-α as a mediator of SREBP-1 maturation was investigated in human hepatocytes.

One significant aspect of the above Examples and discussion is that ceramide stimulated SREBP-1 maturation even in the presence of cholesterol and 25-hydroxycholesterol both of which are known suppresscrs of SREBP-1 maturation. This indicates that ceramide mediated maturation of SREBP-1 maturation is a novel, sterol independent mechanism by which cholesterol homeostasis may be regulated.

The following materials and methods were used as needed in the above Examples 1-8.
1. *Materials-* A continuous line of human hcpatocytes designated lffi-25 were prepared from normal human tissue (18). Alpha modified minimal essential medium was purchased from Mediatech (Herndon, VA). Fetal bovine serum was purchased from Hyclonc, Salt Lake City, Utah. F10 media and the insulin-transferrin-selenium supplement were purchased from Gibco-BRL (Gaithersburg, MD). Human recombinant EGF, PDGF and TNF-α were from Upstate Biotechnology (Lake Placid, NY). C₂-ceramide (N-acetylsphingosine) was obtained from Matrcya (Pleasant Gap, PA). [¹⁴C]-sphingomyelin (specific activity 50mCi/mmol) was from American Radiolabeled Chemicais (St. Louis, MO). Anti-SREBP-1 antibody was purchased from Santa Cruz Biotechnology (Santa Cruz, CA). Sphingomyelinase from Streptomyces species and all other redrugs were obtained from Sigma.
2. *Cell Culture-* HH-25 cells were grown in alpha-minimal essential media supplemented with 100units/ml penicillin, 100 g/ml streptomycin, 10 g/ml insulin, 0.1µM selenium, 5.5 µg/ml transferrin, 0.5 µg/ml linoleic acid and 10% fetal bovine serum (media A). The cells were incubated in serum free F10 media for 30 to 60 minutes prior to initiating treatment with TNF-α, C₂-ceramide or sphingomyelinase.
3. *Cell fractionation-* Following treatment, the cells were washed with 5ml of PBS and pelleted at 1500xg for 10 minutes at 4°C. The pellet was stored at -70°C and lysed in 0.5ml buffer A (10mM HEPES pH 7.4, 5mM EDTA, 025mM EGTA, 50mM NaF, 7mM β-mercaptoethanol, 0.35M sucrose, 0.1% NP-40 and protease inhibitors 1mM PMSF, 2µg/ml aprotinin, 10µg/ml leupeptin and 5µg/ml pepstatin). The lysate was centrifuged at 12,000 x g for 15 minutes at 4°C to prepare a nuclear fraction. The protein concentration of these samples was determined by the method of Lowry et al. al. (19).
*4. Neutral Sphingomyelinase Assay*-After stimulation with TNF-α for the indicated time intervals, the cells were washed once with 5ml PBS and harvested. The pellet was stored frozen at -70°C and resuspended in 0.5ml buffer B (100mM Tris HCl pH 7.4, 0.1 % triton X-100, 1mM EDTA and protease inhibitors). The cell suspension was sonicated 3 times (3 second bursts) using a probe sonicator and centrifuged at 500xg at 4°C for 5 minutes. The supcmatant was used as the enzyme source.
   100 µg of protein was assayed for neutral sphingomyelinase activity in a buffer consisting of 50mM Tris HCl pH 7.4, 0.1% triton X-100, 0.1mg BSA, 5mM MgCl₂, and 50 moles [¹⁴C] sphingomyclin (12,000 dpm). The assay was incubated at 37°C for 1.5 hours and terminated with the addition of 1ml of 10% TCA. The precipitate was pelleted (1000xg at 4°C for 20 minutes) and 1ml of the supernatant was extracted with 1 ml anhydrous diethyl ether. 0.5ml of the aqueous phase was removed for liquid scintillation counting.
5. *Immunoblotting*- 50µg of nuclear protein was separated by gel electrophoresis on a 7.5% polyacrylamide geL Gels were calibrated by high range molecular weight markers (Bio-Rad product #161-0303, New York, NY) which were transferred to a polyvinyl diflouride (PVDF) membrane and visualized with coomassie staining. Rabbit polyclonal antibodies against SREBP-1 were used at 0.5 µg/ml according to the instructions of the manufacturer. The antibody was visualized with horseradish peroxidase conjugated anti-rabbit IgG made in donkey (Amersham) using the Enhanced Chemiluminescence (ECL) Western Blotting Detection System Kit (Amersham). PVDF membranes were exposed to Hyperfilm ECL (Amersham) for the indicated time. Immunoblots were quantified via densitometry performed on a PDI densitometer scanner (model 20J7) coupled to a SPARC IRC workstation.
*5. Indirect Immunofluorescence*-Cultured HH-25 cells were grown on coverslips and treated as described. After treatment, the cells were washed 3 X 5 minutes with PBS containing 1mM MgCl₂ and 0.1mM CaCl₂ (solution A). The cells were fixed with 3% paraformaldehyde in solution A for 10 minutes and permeabilized with 0.5% Triton X-100 in solution A for 6 minutes at room temperature. The coverslips were then washed 3 x 5 minutes with solution A.
   Primary antibody (anti-SREBP1) was used at a dilution of 0.5 g/ml in PBS and applied for 1 hour with gentle shaking. The cells were washed as above and a FITC conjugated anti-rabbit IgG secondary antibody, was applied for 1/2 hour according to the instructions of the manufacturer. The coverslips were washed, mounted on microscope slides and were viewed and photographed at the indicated magnification on a Zeiss Axiovert 25 fluorescence microscope.
6. *DNA laddering assay-* Cells were treated with either TNF-α, sphingomyelinase or C₂-ceramide for 1 hour at concentrations identical to those used in the SREBP-1 maturation studies. The cells were then washed twice with minimal essential medium and refed with media A for 6 hours. The cells were harvested and genomic DNA was prepared as described (22). Genomic DNA was electrophoresed and stained with ethidium bromide.
7. *Electrophoretic Mobility Shift Assays-* Gel mobility shift assays were performed as follows. Each 20 µl reaction mixture contained 8-10 µg of nuclear protein plus a α-[³²P]-labeled 25-base pair oligonucleotide probe containing the SREBP-binding site (14) in binding buffer (10 mM Hepes, pH 7.5, 0.5 mM spamidine, 0.15 mM EDTA, 10 mM dithiothreitol, 0.35 mM sucrose). The reaction mixture was incubated at room temperature for 15 min and loaded directly onto a 6.5% polyacrylamide (49:0.6 acrylamide/bisacrylamide) gel in a buffer of 25 mM Tris borate (pH 8.0), 0.25 mM EDTA. In some experiments, antisera specific for SREBP or preimmune sera were added to reaction mixtures to determine the composition of protein-probe complexes. For these "supershift" assays, extracts were incubated with 1 µl of preimmune sera or an equal volume of anti-SREBP antisera at 4°C for 30 min prior to addition of α-[³²P]-labeled probe. In all experiments, proteins were separated by electrophoresis at 200 V for 2 h at room temperature. Gels were dried and exposed to Kodak XAR film with intensifying screens. Assays were repeated with nuclear extracts obtained from three unique experiments and evaluated by phosphoimage analysis to ensure reproducibility of results.

### References

1) Goeddel, D.V., Aggarwal, B.B., Gray, P.W., Leung, D.W., Nedwin, G.E., Palladino, M.A., Patton, J.S., Pennica, D., Shepard, H.M., Sugarman, BJ. and Wong, G.H.W. (1986) *Cold Spring Harbor Symp. Quant*. *Biol*. **51**, 597-609.
2) Baringa, M., (1996) *Science* 273, 735-737. Bazzoni, F. and Beutler, B. (1996) *NEJM* **334,** 1717-1725
3) Chatterjee, S., (1993) *Adv. Lipid Res*. **26**, 25-48.
4) Tepper, C.G., Jayadev, S., Liu, B.,Bielawska, A., Wolff, R., Yonehara, S., Hannun, Y.A., and Seldin, M.F. (1995) *Proc. Natl. Acad. Sci. U.S.A.* **92**, 8443-8447.
5) Cifone, M.G., DeMaria, R., Roneaioli, P., Rippo, M.R. Azuma, M. Lanier. L.L., Santoni, A., and Testi, R. (1994) *J. Exp. Med.* **180**, 1547-1552.
6) Okazski, T., Bell, R.M., and Hanmun, Y.A. (1995) *J. Biol. Chem.* **264**, 19076-19080.
7) Mathias, S., Younes, A., Kan, C.C., Oriow, L, Joseph, C., and Kolesnick, R.N., (1993) *Science* **259**, 519-522.
8) Dobrowsky, R.T., Werner, M.H., Castellino, A.M., Chao, M.V. and Hannun, Y.A. (1994) *Science* **265**, 1596-1599.
9) Chan, C.G., and Ochi, A. (1995) *Eur. J. Immunol.* **25**, 1999-2004.
10) Kim, M.Y., Linardic, C., and Hamlun, Y.A. (1991) *J. Biol. Chem.* **266**, 484-489.
11) Cuvillier, O., Pirianov, G., Kleuser, B., Vanek, P.G., Coso, O.A., Gutkind, J.S., and Spiegel, S., *Nature* **381,** 800-803.
12) Goldstein, J.L., and Brown, M.S. (1986) *Nature* **343,** 425-430.
13) Dawson, PA, Hofmann, S.L., van der Westhhuyzen, D.R., Brown, M.S., and Goldstein, J.L. (1988) *J. Biol, Chem*. **263**, 3372-3379.
14) Wang, X., Sato, R, Brown, M.S., and Goldstein, J.L. (1994) *Cell* **77,** 53-62.
15) Chatterjee, S., (1994) *J. Biol. Chem*, **269,** 879-882.
16) Chatterjee, S. (1993) *J. Biol. Chem.* **268,** 3401-3406.
17) Hamanaka, R., Kohno, K., Seguchi, T., Okamutra, K., Morimoto, A., Ono, M., Ogata, J., and Kuwano, M. (1992) *J. Biol. Chem*. **267,** 13160-13165.
18) Wang, X., Zelenski, N.G., Yang, J., Sakai, J., Brown, M.S., and Goldstein, J.L. (1996) *EMBO* **15**, 1012-1020.
19) Mizushima, N., Koike, R., Kohsaka, H., Kushi, Y., Handa, S., Yagita, H., Miyasaka N. (1996) *FEBS Lett.* **395,** 267-271.
20) Bittman, R., Kasireddy, C.R., Mattjus, P., and Slotte, J.P. (1994) *Biochemistry* **33**, 11776-11781.
21) Kan, C., Ruan, Z., and Bittman, R., (1991) *Biochemistry* **30**, 10746-10754.
22) Clejan, S., and Bittman, R., (1984) *J. Biol. Chem.* **259**, 10823-10826.
23) Adam-Klages, S., Adam, D., Weigmann, K., Struve, S., Kolanus, W., Schneider-Mergener, J., and Krouke, M. (1996) *Cell,* **86**, 937-947.
24) Lawler, F. J. et al. (1998) *J. Biol. Chem.* **273**: 5058.
25) Shimomura, L, et al. (1998) *J. Biol. Chem.* **273**; 35299.
26) Brown, M. S. and J. L. Goldstein (1997) *Cell* **89**: 331.
27) Frishman W,H, and Rapier, R.C., (1989) Med. clinics of North America **73**, 437-448.

The invention has been described with reference to preferred embodiments thereof. However, it will be appreciated that those skilled in the art, upon consideration of this disclosure, may make modifications and improvements.

## Claims

1. An anti-lipemic drug comprising ceramide, which is an effector of the sterol regulatory element binding protein-1 (SREBP-1), and a serum cholesterol inhibitor, which is an inhibitor of HMG CoA reductase.

2. Anti-lipemic drug according to claim 1, wherein the ceramide is C-2 ceramide.

3. Anti-lipemic drug according to claim 1 or 2, wherein the serum cholesterol inhibitor is selected from the group comprising fluvastatin, simvastatin, lovastatin, pravastatin, mevinolin and atorvastatin.

4. Use of an anti-lipemic drug as defined in claims 1-3 for the preparation of a pharmaceutical composition for modulating serum cholesterol level in a mammal, and/or for treating a disorder in a mammal associated with high serum cholesterol levels.

5. An *ex vivo* method for determining therapeutic capacity of an anti-lipsmic drug as defined in claims 1-3 for treating a cholesterol related disease in a mammal, the method comprising:
a) providing a population of cells capable of expressing SREBP-1,
b) contacting the cells with the anti-lipemic drug in an amount sufficient to induce maturation of the SREBP-1,
c) culturing the cells in medium: and
d) detecting maturation of the SREBP-1 as indicative of the therapeutic capacity of the anti-lipemic drug in treating the disease.

## Patentansprüche

1. Antilipämisches Medikament, das Ceramid, bei dem es sich um einen Effektor des sterolregulierendes Element bindenden Proteins 1 (SREBP-1) handelt, und einen Serumcholesterin-Inhibitor, bei dem es sich um einen Inhibitor der HMG-CoA-Reductase handelt, umfasst.

2. Antilipämisches Medikament gemäß Anspruch 1, wobei es sich bei dem Ceramid um C-2-Ceramid handelt.

3. Antilipämisches Medikament gemäß Anspruch 1 oder 2, wobei der Seruniclioiesterin-Inhibitor aus der Gruppe ausgewählt ist, die Fluvastatin, Simvastatin, Lovastatin, Pravastatin, Mevinolin und Atorvastatin umfasst.

4. Verwendung eines antilipämischen Medikaments gemäß den Ansprüchen 1 bis 3 zur Herstellung einer pharmazeutischen Zusammensetzung zum Modulieren des Serumcholesterinspiegels bei einem Säuger und/oder zur Behandlung einer Störung bei einem Säuger, die mit hohen Serumcholesterinspiegeln verbunden ist.

5. Ex-vivo-Verfahren zur Bestimmung der therapeutischen Kapazität eines antilipämischen Medikaments gemäß den Ansprüchen 1 bis 3 zur Behandlung einer mit Cholesterin zusammenhängenden Krankheit bei einem Säuger, wobei das Verfahren Folgendes umfasst;
a) Bereitstellen einer Population von Zellen, die SREBP-1 exprimieren können;
b) In-Kontakt-Bringen der Zellen mit dem antilipämischen Medikament in einer ausreichenden Menge, um die Reifung des SREBP-1 zu induzieren;
c) Kultivieren der Zellen in Medium; und
d) Nachweisen der Reifung des SREBP-1 als Indikator der therapeutischen Kapazität des antilipämischen Medikaments bei der Behandlung der Krankheit.

## Revendications

1. Un médicament à action anti-lipémique comprenant une céramide consistant en un effecteur de la protéine-1 se liant à l'élément régulateur du stérol (SREBP-1), et un sérum inhibiteur du cholestérol, consistant en un inhibiteur de la HMG CoA réductase.

2. Médicament à action anti-lipémique selon la revendication 1, dans lequel la céramide est la C-2 céramide.

3. Médicament à action anti-lipémique selon la revendication 1 ou 2, dans lequel l'inhibiteur du cholestérol est choisi dans le groupe comprenant fluvastatine, simvastatine, lovastatine, pravastatine, mévinoline et atorvastatine.

4. Utilisation d'un médicament anti-lipémique tel que défini dans les revendications 1 à 3, pour la préparation d'une composition pharmaceutique pour moduler le niveau de cholestérol chez un mammifère et/ou pour traiter un désordre associe à un taux élevé en cholestérol d'un mammifère.

5. Un procédé ex-vivo pour déterminer la capacité thérapeutique d'un médicament anti-lipémique tel que défini dans les revendications 1 à 3, pour traiter un désordre provoqué par le cholestérol chez un mammifère, ce procédé comprenant :
a) l'utilisation d'une population de cellules capables d'exprimer SREBP-1 ;
b) le contact des cellules avec un médicament à action anti-lipémique en une quantité suffisante pour induire la maturation de SREBP-1 ;
c) la culture des cellules dans le milieu ; et
d) la détection de la maturation du SREBP-1 comme indication de la capacité thérapeutique du médicament à action anti-lipémique pour le traitement dudit désordre.
